# EUROPÄISCHE PATENTSCHRIFT

(11) **EP 3 610 908 B1**
(45) Veröffentlichungstag und Bekanntmachung des Hinweises auf die Patenterteilung: **30.12.2020**
(21) Anmeldenummer: 19196409.7
(22) Anmeldetag: 13.07.2015
(51) Int. Cl.: A61M 5/20, A61M 5/31, A61M 5/315, A61M 5/32

(54) **AUTOINJEKTOR MIT BEWEGBAREM NADELSCHUTZ**
AUTOINJECTOR WITH MOVABLE NEEDLE PROTECTOR
AUTO-INJECTEUR AVEC PROTECTEUR D'AIGUILLE MOBILE

(30) Priorität: 29.07.2014 CH 11612014
(43) Veröffentlichungstag der Anmeldung: 19.02.2020
(62) Dokumentnummer(n) der früheren Anmeldung(en) nach Art. 76 EPÜ: 15739500.5 / 3 174 578
(73) Patentinhaber: Ypsomed AG, 3401 Burgdorf (CH)
(72) Erfinder: Tschirren, Markus, 3400 Burgdorf (CH); Hirschel, Jürg, 3007 Bern (CH); Schrul, Christian, 3400 Burgdorf (CH)
(74) Vertreter: Kleiner, Stefan

(56) Entgegenhaltungen:
- EP-A1- 2 489 380
- EP-A2- 2 742 962
- WO-A1-2010/081489
- WO-A1-2013/016832
- CH-A2- 707 216
- US-A1- 2005 261 634

## Beschreibung

Die Erfindung betrifft einen Autoinjektor zur Verabreichung einer einstellbaren Dosis eines flüssigen Produkts, insbesondere eines Medikaments. Der Autoinjektor ist vorzugsweise so gestaltet, dass mit ihm eine Dosiseinstellung und nur eine einzige Produktausschüttung möglich bzw. der Autoinjektor nach der einzigen Produktausschüttung in einem Zustand ist, der eine weitere Produktausschüttung verhindert. Eine nicht-ausgeschüttete Produktmenge verbleibt in dem Autoinjektor. Solche Autoinjektoren können als Einzelschussautoinjektor bezeichnet werden (engl. single-shot autoinjection device).

Der Begriff "Medikament" umfasst hier jede fliessfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. "Medikament" kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oglionukleotide, Antikörper oder Teile von Antikörpern sowie geeignete Basis-, Hilfs- und Trägerstoffe.

Aus dem Stand der Technik sind Autoinjektoren bekannt. Aus der EP 2 742 962 A2 ist ein Autoinjektor nach dem Oberbegriff des Anspruchs 1 bekannt. Der Autoinjektor aus der EP 2 742 962 A2 ist so gestaltet, dass er die in seinem Produktbehälter enthaltene Produktmenge mit einer einzelnen Injektion nur vollständig ausstossen kann.

Für bestimmte Therapien kann es erforderlich sein, nur eine bestimmte Menge des in dem Produktbehälter enthaltenen Produkts auszustossen. Ein Ansatz wäre, den Produktbehälter nur mit der gewünschten Produktdosis zu füllen, so dass die vollständige Ausschüttung des Produkts der Ausschüttung der gewünschten Produktdosis entspricht. Allerdings müssten dann zum Beispiel in einer Apotheke verschiedene Autoinjektoren mit dem gleichen Medikament jedoch mit unterschiedlichen Füllmengen ihrer Produktbehälter vorgehalten werden. Einfacher ist es, einen Autoinjektor bereitzustellen, der mit einer grösseren Produktmenge gefüllt ist und der angepasst ist, je nach Wunsch nur einen Teil dieser Produktmenge auszustossen, wobei der Autoinjektor einschliesslich der im Autoinjektor verbleibenden Restmenge des Produkts entsorgt wird.

Es ist eine Aufgabe der Erfindung, einen Autoinjektor bereitzustellen, der die Einstellung einer auszuschüttenden Produktdosis ermöglicht.

Die Aufgabe wird mit einem Autoinjektor mit den Merkmalen des Anspruchs 1 gelöst. Vorteilhafte Weiterbildungen ergeben sich aus den abhängigen Ansprüche, der Beschreibung und den Figuren.

Die Erfindung geht von einem Autoinjektor zur Verabreichung eines flüssigen Produkts, insbesondere Medikaments aus, wobei der Autoinjektor umfasst:
- ein Gehäuse, welches vorzugsweise hülsenförmig und länglich mit einer Längsachse gebildet ist,
- einen Produktbehälter, insbesondere eine Spritze, an dessen distalem Ende eine Nadel, insbesondere lösbar oder unlösbar angeordnet ist, in dem ein Kolben, der vorzugsweise dichtend an der ihn umgebenden Wand des Produktbehälters verschiebbar angeordnet ist und der in einem Produktbehälterhalter, der auch als Spritzenhalter bezeichnet werden kann, gehalten ist, wobei der Produktbehälterhalter axialfest mit dem Gehäuse vorzugsweise permanent verbunden, insbesondere verschnappt oder formschlüssig verbunden ist, wobei die Nadel aus dem Gehäuse über das distale Ende des Gehäuses ragt oder hervorsteht,
- eine Nadelschutzhülse, die insbesondere die Funktion einer Nadelschutzhülse und einer Auslöseeinrichtung für die Produktausschüttung inne hat, wobei die Nadelschutzhülse aus einer Ausgangsposition, in der das distale Ende der Nadelschutzhülse distal über die Nadelspitze der Nadel steht, so dass insbesondere der Zugriff auf die Nadel verhindert wird, in das Gehäuse verschiebbar ist, insbesondere in die proximale Richtung, so dass die Nadel aus dem distalen Ende der Nadelschutzhülse hervortritt, insbesondere mit einer Länge hervortritt, die in etwa der Einstechtiefe der Nadel, vorzugsweise für eine subkutane Injektion, entspricht, und
- einen Stössel und eine Ausschüttfeder, wobei bevorzugt ist, dass die Ausschüttfeder eine als Druckfeder wirkende Wendelfeder ist, wobei der Stössel in dem Gehäuse angeordnet ist und von der insbesondere im Auslieferungszustand des Autoinjektors vorgespannten Ausschüttfeder, welche vorzugsweise zumindest teilweise innerhalb des vorzugsweise hülsenförmigen Stössels angeordnet ist, entlang einer Längsachse des Autoinjektors oder des Gehäuses in die distale Richtung verschiebbar ist, wobei die Verschiebung des Stössels in die distale Richtung bewirkt, dass der Kolben, an dem der Stössel zumindest bei der Verschiebung anstösst, von dem Stössel mitgenommen wird und das Produkt aus dem Produktbehälter, insbesondere über die Nadel, verdrängt.

Der Autoinjektor umfasst ferner ein Dosiseinstellelement, welches vorzugsweise eine Aussenfläche des Autoinjektors bildet und von dem Verwender des Autoinjektors greifbar ist, wobei das Dosiseinstellelement zur Einstellung einer aus dem Produktbehälter auszuschüttenden Dosis des Produkts relativ zu dem Gehäuse, insbesondere durch Muskelkraft des Verwenders, verdrehbar ist und mindestens zwei verschiedene, insbesondere durch Rastpositionen vorgegebene vorzugsweise quasistabile Drehpositionen relativ zu dem Gehäuse einnehmen kann. Unter einer quasistabilen Drehposition wird verstanden, dass das Dosiseinstellelement relativ zu dem Gehäuse nur mit einem erhöhten Drehmoment aus der jeweiligen quasistabilen Drehposition drehbar ist, wobei das erhöhte Drehmoment zum Lösen der quasistabilen Drehposition deutlich höher ist als die Gleit- und/oder Haftreibung zur Drehung des Dosiseinstellelements zwischen den mindestens zwei vorgegebenen Drehpositionen.

Der Autoinjektor weist ferner eine Dosierhülse auf, wobei eines aus Dosierhülse und Stössel einen Dosisauswahlanschlag und das andere aus Dosierhülse und Stössel mindestens einen Dosieranschlag aufweist, wobei die Drehung des Dosiseinstellelements bewirkt, dass eines aus Dosierhülse und Stössel relativ zu dem anderen aus Dosierhülse und Stössel verdreht wird. Dasjenige Teil, welches verdreht wird, ist insbesondere unmittelbar oder mittelbar, d. h. über weitere Teile des Autoinjektors, mit dem Dosiseinstellelement verbunden oder gekoppelt. Zum Beispiel kann die Drehung des Dosiseinstellelements eine Drehung der Dosierhülse relativ zu dem Stössel bewirken. Alternativ kann die Drehung des Dosiseinstellelements eine Drehung des Stössels relativ zu der Dosierhülse bewirken. Das Teil, welches bei der Drehung des Dosiseinstellelements nicht gedreht wird, kann zum Beispiel unmittelbar oder mittelbar, d. h. über mindestens ein weiteres Teil, mit dem Gehäuse des Autoinjektors drehfest verbunden sein.

In einer ersten Drehposition der mindestens zwei Drehpositionen sind der Dosisauswahlanschlag und ein erster Dosieranschlag des mindestens einen Dosieranschlags in einer Flucht entlang der Längsachse angeordnet. In einer Flucht bedeutet insbesondere, dass der Dosisauswahlanschlag und der entsprechende Dosieranschlag sich entlang der Längsachse gegenüberliegen und mit einer rein translatorischen Bewegung entlang der Längsachse aneinander anstossen können.

In einer zweiten Drehposition der mindestens zwei Drehpositionen ist der Dosisauswahlanschlag um die Längsachse winkelversetzt zu dem ersten Dosieranschlag angeordnet. D. h. insbesondere, dass für den Winkelversatz die Längsachse die Drehachse für den Drehwinkel bildet. Grundsätzlich können mindestens drei, vier oder noch mehr Drehpositionen vorgesehen sein.

Die Verschiebung der Nadelschutzhülse in das Gehäuse bewirkt, dass die Ausschüttfeder den Stössel in die distale Richtung verschiebt, insbesondere eine Freigabe eines den Stössel blockierenden Eingriffs, der die Bewegung des Stössels in die distale Richtung verhindert, wodurch in Abhängigkeit der Drehposition des Dosiseinstellelements verschieden grosse Produktmengen ausgebbar sind bzw. ausgegeben werden. Wenn das Dosiseinstellelement in seiner ersten Drehposition ist, wird einer aus Dosisauswahlanschlag und erstem Dosieranschlag zu dem anderen, zum Beispiel in Bezug auf das Gehäuse ruhenden, aus Dosisauswahlanschlag und erstem Dosieranschlag hin bewegt. Einer aus Dosisauswahlanschlag und erstem Dosieranschlag stösst somit an dem anderen aus Dosisauswahlanschlag und erstem Dosieranschlag an, insbesondere derjenige, der sich zu dem anderen hin bewegt.

Wenn das Dosiseinstellelement in seiner zweiten Drehposition ist, wird einer aus Dosisauswahlanschlag und erstem Dosieranschlag an dem anderen aus Dosisauswahlanschlag und erstem Dosieranschlag entlang der Längsachse vorbeibewegt, oder ist vorbeibewegbar. Hierdurch kann der Stössel einen grösseren Hub ausführen als wenn das Dosiseinstellelement in seiner ersten Drehposition ist, so dass eine grössere Dosis aus dem Produktbehälter ausstossbar ist.

Beispielsweise kann der Stössel den Dosisauswahlanschlag und die Dosierhülse den mindestens einen oder die mehreren Dosieranschläge aufweisen. Alternativ kann der Stössel den mindestens einen Dosieranschlag oder die mehreren Dosieranschläge aufweisen, wobei die Dosierhülse den Dosisauswahlanschlag aufweisen kann.

In einer Ausführung kann das Dosiseinstellelement ein am proximalen Ende des Gehäuses befestigter, von einem Verwender des Autoinjektors greifbarer und mit Muskelkraft verdrehbarer Drehknopf sein, wobei das Dosiseinstellelement in Bezug auf das Gehäuse axialfest und drehbar ist, insbesondere axialfest und drehbar mit dem Gehäuse verschnappt ist.

In einer Weiterbildung kann eine Rasteinrichtung vorgesehen sein, welche die insbesondere quasistabilen Rastpositionen für das Dosiseinstellelement bereitstellt. Die Rasteinrichtung kann zum Beispiel je Rastposition eine Ausnehmung, insbesondere eine Nut am Gehäuse oder einem gehäusefesten Element, und ein Rastelement, welches je nach Drehposition des Dosiseinstellelements in eine der Ausnehmungen eingreift, aufweisen. Das Rastelement kann an dem Dosiseinstellelement oder einen drehfest mit dem Dosiseinstellelement verbundenen Teil befestigt sein, so dass sich das Rastelement mit dem Dosiseinstellelement mitdreht. Alternativ kann die Rasteinrichtung zum Beispiel je Rastposition eine Ausnehmung, insbesondere eine Nut am Dosiseinstellelement, und ein Rastelement, welches je nach Drehposition des Dosiseinstellelements in eine der Ausnehmungen eingreift, aufweisen. Das Rastelement kann an dem Gehäuse oder einem gehäusefesten Element oder Einsatz befestigt sein, so dass sich das Dosiseinstellelement bei der Dosiseinstellung relativ zu dem Rastelement dreht.

In einer Weiterbildung können in der zweiten Drehposition der mindestens zwei Drehpositionen der Dosisauswahlanschlag und ein zweiter Dosieranschlag des mindestens einen Dosieranschlags in einer Flucht entlang der Längsachse angeordnet sein. Einer aus Dosisauswahlanschlag und zweitem Dosieranschlag kann sich zu dem anderen aus Dosisauswahlanschlag und zweitem Dosieranschlag hin bewegen, wobei der andere ruht, wobei insbesondere einer aus Dosisauswahlanschlag und zweitem Dosieranschlag an dem anderen aus Dosisauswahlanschlag und zweitem Dosieranschlag anstösst, wenn das Dosiseinstellelement in seiner zweiten Drehposition ist und die Ausschüttfeder den Stössel in die distale Richtung verschiebt.

In einer Weiterbildung können der erste Dosieranschlag und der zweite Dosieranschlag in Bezug zueinander entlang der Längsachse beabstandet und um die Längsachse winkelversetzt angeordnet sein. Insbesondere kann der Winkelversatz zwischen dem ersten und zweiten Dosieranschlag dem Winkelversatz zwischen der ersten und zweiten Drehposition entsprechen.

In einer Weiterbildung kann der Produktbehälter einen Anschlag, der insbesondere von einem sich verjüngenden Abschnitt zwischen dem zylindrischen Teil des Produktbehälters und der Nadel gebildet sein kann, aufweisen, an dem der Kolben anstösst, wenn das Dosiseinstellelement in seiner zweiten oder einer dritten Drehposition seiner Drehpositionen ist und die Ausschüttfeder den Stössel in die distale Richtung verschiebt. Beispielsweise kann, wenn sich das Dosiseinstellelement in seiner dritten Drehposition befindet, einer aus Dosisauswahlanschlag und zweitem Dosieranschlag an dem anderen aus Dosisauswahlanschlag und zweitem Dosieranschlag entlang der Längsachse vorbeibewegbar sein oder vorbeibewegt werden.

Allgemein kann die Dosierhülse oder ein von der Dosierhülse und dem Produktbehälter verschiedenes Teil des Autoinjektors einen Anschlag aufweisen, an dem der Dosisauswahlanschlag des Stössels oder ein weiterer Dosisauswahlanschlag des Stössels anstösst oder zu dem der Dosisauswahlanschlag hin bewegt wird, wenn das Dosiseinstellelement in einer zweiten oder einer dritten Drehposition seiner Drehpositionen ist und die Ausschüttfeder den Stössel in die distale Richtung verschiebt. Das von der Dosierhülse von dem Produktbehälter verschiedene Teil kann zum Beispiel axialfest mit dem Gehäuse verbunden oder gekoppelt sein oder von dem Gehäuse gebildet werden.

### Erste Ausführungsform

In einer ersten Ausführungsform kann der Dosisauswahlanschlag an dem Stössel gebildet sein, wobei der mindestens eine Dosieranschlag einen ersten Dosieranschlag und einen zweiten Dosieranschlag aufweist, die an der Dosierhülse gebildet sind, wobei die Dosierhülse in Bezug auf das Gehäuse drehfest ist und der Stössel drehfest mit dem Dosiseinstellelement verbunden und zusammen mit dem Dosiseinstellelement in Bezug auf das Gehäuse und/oder die Dosierhülse verdrehbar ist.

In einer Weiterbildung der ersten Ausführungsform kann die Dosierhülse einen Stoppanschlag aufweisen, wobei ein relativ zu dem Gehäuse axialfester und vorzugsweise drehbarer Mechanikhalter, der insbesondere mit dem Gehäuse axialfest und drehbar verschnappt sein kann, ein Stoppgegenanschlag aufweisen kann. Der Dosisauswahlanschlag stösst während der Bewegung des Stössels in die distale Richtung an dem ersten oder zweiten Drehanschlag an, je nach Drehposition des Dosiseinstellelements, und nimmt die Dosierhülse in die distale Richtung mit, wodurch der Stoppanschlag zu dem Stoppgegenanschlag bewegt wird, wobei die Bewegung des Stössels und insbesondere der Dosierhülse in die distale Richtung blockiert ist, wenn der Stoppanschlag an dem Stoppgegenanschlag anstösst.

Eine Weiterbildung zeichnet sich durch ein Verschiebeglied aus, welches ein erstes Eingriffsglied aufweist, das in eine Ausnehmung des Mechanikhalters eingreift, so dass das Verschiebeglied in Bezug auf das Gehäuse axialfest gehalten wird, wobei das erste Eingriffsglied von der Dosierhülse gehindert wird, sich aus der Ausnehmung zu bewegen, wenn der Stoppanschlag von dem Stoppgegenanschlag beabstandet ist, wobei durch die Mitnahme der Dosierhülse durch den Stössel die Dosierhülse aus der Position bewegt wird bzw. bewegbar ist, in der die Dosierhülse das erste Eingriffsglied hindert, sich aus der Ausnehmung zu bewegen. Vorteilhaft wird durch den Ausschütthub die Mitnahme der Dosierhülse bewirkt und dadurch die Blockierung des ersten Eingriffsglieds gelöst.

Eine Weiterbildung zeichnet sich durch eine weitere vorgespannte Feder, die auch als Nadelschutzfeder bezeichnet werden kann, aus, welche insbesondere mit ihrem proximalen Ende auf das Verschiebeglied wirkt und zum Beispiel mit ihrem distalen Ende unmittelbar oder bevorzugt mittelbar, wie z.B. über eine Schalthülse, auf die Nadelschutzhülse wirken kann, wodurch das Verschiebeglied relativ zu dem Gehäuse in die proximale Richtung bewegt wird bzw. bewegbar ist, wobei vorteilhaft:
- das Verschiebeglied während der Bewegung in die proximale Richtung gegen einen feststehenden Teil des Autoinjektors anschlägt und dadurch ein taktiles und/oder akustisches Signal erzeugt, welches das Ende der Ausschüttung der eingestellten Dosis signalisiert, wobei das Verschiebeglied somit als Signalerzeugungsmittel dient, und/oder
- das mit dem Mechanikhalter und dem Dosiseinstellelement drehfest verbundene Verschiebeglied durch die Bewegung in die proximale Richtung in einen drehfesten und insbesondere formschlüssigen Eingriff mit dem Gehäuse oder einem gehäusefesten Element bewegt oder gekuppelt wird, so dass das Dosiseinstellglied in Bezug auf das Gehäuse um die Längsachse drehfest ist, wobei z.B. das Verschiebeglied hierbei als Kupplungsglied wirkt, welches drehfest in den formschlüssigen Eingriff mit dem Gehäuse oder dem gehäusefesten Element gekuppelt wird. Das Verschiebeglied kann sich erst dann in die proximale Richtung bewegen, d. h. das Signal erzeugen und/oder die drehfeste Kopplung bewirken, wenn das erste Eingriffsglied aus der Ausnehmung bewegt wird oder bewegbar ist, d. h., wenn die Dosierhülse bzw. deren Stoppanschlag an dem Stoppgegenanschlag anschlägt.

In einer Weiterbildung nicht nur der ersten Ausführungsform sondern auch des allgemein beschriebenen Autoinjektors kann ein zweites Eingriffsglied, welches vorzugsweise von dem Verschiebeglied gebildet wird, vorgesehen sein, welches in eine Ausnehmung des Stössels lösbar eingreift und dadurch den Stössel an einer Bewegung relativ zu dem Gehäuse in die distale Richtung blockiert, wobei in einer (Ausgangs-)Drehposition des Dosiseinstellelements eine Haltefläche des Gehäuses oder eines gehäusefesten Elements das zweite Eingriffsglied hindert, sich aus der Ausnehmung zu bewegen und in der ersten und/oder zweiten Drehposition oder einer Primingdrehposition des Dosiseinstellelements die Haltefläche und das zweite Eingriffsglied mit einem Drehwinkel um die Längsachse beabstandet sind, so dass das zweite Eingriffsglied aus der Ausnehmung des Stössels bewegbar ist und der Stössel zum Ausführen eines Priminghubs von der Ausschüttfeder in die distale Richtung bewegbar ist. Der Priminghub dient dazu, etwaig in dem Produktbehälter enthaltene Luft aus dem Produktbehälter zu verdrängen und das fluidführende System bis zur Nadelspitze hin mit dem flüssigen Produkt zu füllen. Dadurch wird vermieden, dass dem Patienten Luft injiziert wird, was vermieden werden sollte.

In einer Weiterbildung kann der Stössel am Ende des Priminghubs an ein in eine Ausnehmung des Stössels eingreifendes erstes Eingriffselement anschlagen, wobei bevorzugt ist, dass das erste Eingriffselement von einem Halteelement gebildet wird, an dem sich das proximale Ende der Ausschüttfeder abstützt, wobei das erste Eingriffselement eine Bewegung des Stössels in die distale Richtung lösbar blockiert, wobei die Nadelschutzhülse, wenn sie in das Gehäuse bewegt wird, eine Sperrhülse aus einer Position, in der die Sperrhülse verhindert, dass das erste Eingriffselement aus der Ausnehmung des Stössels bewegbar ist, in die proximale Richtung in eine Freigabeposition verschiebt, wobei die Sperrhülse eine Ausnehmung aufweist, die in der Freigabeposition entlang der Längsachse in etwa mit der Position eines zweiten Eingriffselements des Halteelements, wobei das zweite Eingriffselement an dem ersten Eingriffselement vorzugsweise gebildet ist, übereinstimmt, so dass das zweite Eingriffselement in die Ausnehmung und das erste Eingriffselement gleichzeitig aus dem Eingriff mit dem Stössel bewegbar ist, wodurch die Bewegung des Stössels in die distale Richtung für die Ausschüttung der eingestellten Dosis freigegeben ist.

### Zweite Ausführungsform

Eine zweite Ausführungsform des Autoinjektors zeichnet sich dadurch aus, dass der Dosisauswahlanschlag an dem Stössel gebildet ist, wobei der mindestens eine Dosieranschlag einen ersten Dosieranschlag und einen zweiten Dosieranschlag umfasst, die an der Dosierhülse gebildet sind, wobei die Dosierhülse in Bezug auf das Gehäuse drehfest ist, insbesondere drehfest in das Gehäuse oder in ein gehäusefestes Teil eingreift, wobei der Stössel drehfest mit dem Dosiseinstellelement verbunden oder gekoppelt und zusammen mit dem Dosiseinstellelement in Bezug auf das Gehäuse und/oder die Dosierhülse verdrehbar ist. Eine Drehung des Dosiseinstellelements bewirkt eine Drehung des Dosisauswahlanschlags relativ zu dem ersten und zweiten Dosieranschlag. Selbstverständlich können mehr als zwei Dosieranschläge, wie zum Beispiel acht Dosieranschläge oder noch mehr an der Dosierhülse vorgesehen sein.

Beispielsweise kann die Dosierhülse axialfest mit dem Gehäuse verbunden sein, insbesondere verschnappt sein. Alternativ kann die Dosierhülse verschiebbar mit dem Gehäuse verbunden sein, wobei sich die Dosierhülse zur axialen Fixierung am proximalen Ende des Spritzenkörpers des Produktbehälters abstützen kann.

In einer Weiterbildung kann die Dosierhülse ein insbesondere flexibles und/oder quer zur Längsachse bewegbares Eingriffselement aufweisen, welches in eine insbesondere kulissenartig gebildete erste Ausnehmung des Stössels eingreift, wobei die Ausnehmung eine erste Anschlagfläche aufweist, die an dem Eingriffselement anliegt, wodurch eine Bewegung des Stössels in die distale Richtung relativ zu dem Gehäuse und/oder der Dosierhülse blockiert ist. Die erste Anschlagsfläche kann durch Drehung des Dosiseinstellelements und insbesondere der dadurch auf den Stössel übertragenen Drehung aus der Anlage mit dem Eingriffselement gedreht werden oder drehbar sein, wodurch die axiale Blockierung des Stössels freigegeben ist und der Stössel mittels der Antriebsfeder um einen Priminghub in die distale Richtung verschiebbar ist. Am Ende des Priminghubs liegt das Eingriffselement an einer zweiten vorzugsweise ringförmig um den Stössel umlaufenden Anschlagfläche, die von der Ausnehmung gebildet wird, an. Der entlang der Längsachse bestehender Abstand zwischen der ersten und zweiten Anschlagfläche entspricht im Wesentlichen dem Priminghub. Wenn das Eingriffselement an der zweiten Anschlagfläche anliegt, ist die Bewegung des Stössels in die distale Richtung blockiert.

In einer Weiterbildung kann die Nadelschutzhülse, wenn sie in das Gehäuse, insbesondere mittels einer Bewegung in die proximale Richtung und/oder um einen Betätigungshub bewegt wird, eine Sperrhülse aus einer Position, in der sie verhindert, dass das Eingriffselement aus der Ausnehmung des Stössels bewegbar ist, in die proximale Richtung in eine Freigabeposition verschoben werden, wobei die Sperrhülse in der Freigabeposition eine Bewegung des Eingriffselements aus des Ausnehmung, insbesondere eine Bewegung des Eingriffselements quer zur Längsachse, frei gibt, wodurch die Bewegung des Stössels in die distale Richtung für die Ausschüttung der eingestellten Dosis, insbesondere die Bewegung des Stössels für einen Ausschütthub, freigegeben ist.

In einer Weiterbildung kann der Stössel drehfest mit dem Dosiseinstellelement gekoppelt sein, wenn das Eingriffselement an dem ersten Anschlag oder/und dem zweiten Anschlag der Ausnehmung anliegt, und rotatorisch von dem Dosiseinstellelement
- während der Ausschüttung der Dosis entkoppelt werden oder
- entkoppelt sein, wenn der Dosisauswahlanschlag an dem ausgewählten Dosieranschlag, insbesondere dem ersten oder zweiten Dosieranschlag anliegt. Hierdurch wird das Dosiseinstellelement rotatorisch von dem Stössel entkoppelt, so dass von dem Dosiseinstellelement auf den Stössel kein Drehmoment mehr übertragbar ist.

In einer Weiterbildung zeichnet sich der Autoinjektor durch ein Verschiebeglied aus, welches drehfest und axial verschiebbar in Bezug auf die Dosierhülse ist, insbesondere die Dosierhülse umgibt und/oder drehfest oder axialfest verschiebbar in die Dosierhülse eingreift, und einen um die Längsachse drehbaren Ring, welcher vorzugsweise die Dosierhülse umgibt, insbesondere an der Dosierhülse gelagert ist. Der drehbare Ring ist mit der Dosierhülse in einem durch Drehung lösbaren axialfesten Eingriff, zumindest in eine Richtung entlang der Längsachse vorzugsweise in die proximale Richtung. Der Stössel, insbesondere dessen Dosisauswahlanschlag ist ausgestaltet, das Verschiebeglied gegen Ende des Ausschütthubs mitzunehmen, d. h. kurz vor dem Erreichen eines der Dosieranschläge bzw. des ausgewählten Dosieranschlags. Insbesondere kann der Dosisauswahlanschlag während des Ausschütthubs an dem Verschiebeglied anschlagen und dieses mitnehmen, bis der Dosisauswahlanschlag an einem der Dosieranschläge bzw. an dem ausgewählten Dosieranschlag anstösst. Das Verschiebeglied weist eine zum Beispiel abgeschrägte Getriebefläche auf, die an einer zum Beispiel abgeschrägten Getriebegegenfläche des Rings oder allgemein an dem Ring abgleitet, wodurch der Ring um die Längsachse gedreht und aus dem axialfesten Eingriff, in dem der Ring mit der Dosierhülse steht, gedreht wird, so dass eine vorgespannte Feder, insbesondere Kupplungs- oder Klickfeder, den Ring in die proximale Richtung verschiebt oder verschieben kann.

Insbesondere kann die Feder den Ring gegen einen axial feststehenden Teil des Autoinjektors, insbesondere eine Kupplungshülse, beschleunigen, wodurch beim Auftreffen auf das axial feststehende Teil ein akustisches und/oder taktiles Signal erzeugt wird, welches das Ende der Ausschüttung der ausgewählten Dosis signalisiert. Alternativ oder zusätzlich kann die Feder den Ring in einen drehfesten Eingriff, insbesondere in eine sich entlang der Längsachse erstreckende Nut der Dosierhülse verschieben, wodurch der Ring drehfest mit der Dosierhülse und/oder dem Gehäuse gekoppelt wird. Ferner bewirkt die Verschiebung des Rings durch die Feder, dass der Ring eine Position einnimmt, in der der Ring drehfest in die Kupplungshülse eingreift, die ihrerseits drehfest mit dem Dosiseinstellelement gekoppelt ist, so dass insbesondere die Drehung des Dosiseinstellelements relativ zu dem Gehäuse gesperrt wird. Zum Beispiel kann der Ring zusammen mit der Kupplungshülse eine Klauenkupplung bilden, die mit der Bewegung des Rings in die proximale Richtung in den drehfesten Kupplungseingriff kuppelbar ist.

Die Kupplungshülse ist vorzugsweise in einem insbesondere lösbaren Kupplungseingriff mit dem Stössel, wenn das Eingriffselement an dem ersten Anschlag und/oder dem zweiten Anschlag der Ausnehmung anliegt, wobei der Kupplungseingriff während der Ausschüttung der Dosis gelöst wird oder gelöst ist, wenn der Dosisauswahlanschlag an dem ausgewählten Dosieranschlag anliegt.

### Dritte Ausführungsform

Eine dritte Ausführungsform des Autoinjektors zeichnet sich dadurch aus, dass der Dosisauswahlanschlag an der Dosierhülse gebildet ist, wobei der mindestens eine Dosieranschlag zum Beispiel einen ersten Dosieranschlag und optional einen zweiten Dosieranschlag umfasst, wobei der Dosieranschlag oder die Dosieranschläge an einem Stössel gebildet sind, wobei der Stössel in Bezug auf das Gehäuse drehfest ist, wobei die Dosierhülse drehfest mit dem Dosiseinstellelement verbunden oder gekoppelt und zusammen mit dem Dosiseinstellelement in Bezug auf das Gehäuse und/oder dem Stössel verdrehbar ist, wobei die Dosierhülse beispielsweise axialfest mit dem Gehäuse verbunden ist.

Die Dosierhülse kann einen Hauptabschnitt, einen Federabschnitt und distal des Hauptabschnitts entlang der Längsachse mittels des Federabschnitts federnd angeordneten Abschnitt aufweisen. Der Hauptabschnitt oder der federn angeordnete Abschnitt kann den Dosisauswahlanschlag bilden.

In einer Weiterbildung kann eine Verdrehsicherungshülse, welche den Stössel und das Gehäuse verdrehgesichert verbindet, vorgesehen sein, wobei die Verdrehsicherungshülse zwischen dem federnd angeordneten Abschnitt der Dosierhülse und einem proximalen Ende des Produktbehälters, insbesondere einem Fingerflansch angeordnet ist, so dass der Federabschnitt der Dosierhülse den Produktbehälter über den Verdrehsicherungsabschnitt, der vorzugsweise relativ zu dem Gehäuse axial verschiebbar ist, in einem festen Sitz mit dem Produktbehälterhalter drückt.

Im Folgenden werden für die ersten bis dritten Ausführungsformen bevorzugte Ausgestaltungen anhand von Figuren beschrieben. Die dabei offenbarten Merkmale bilden den Gegenstand der Erfindung jeweils einzeln und in jeglicher Merkmalskombination vorteilhalft weiter. Es zeigen:
- Figur 1: eine Explosionsdarstellung eines Autoinjektors gemäss einer ersten Ausführungsform,
- Figuren 2a-2c: die erste Ausführungsform in einem Auslieferungszustand, wobei die Figuren 2a bis 2c durch die Längsachse der Vorrichtung verlaufende Schnittansichten sind, wobei die Schnittanschichten um die Längsachse winkelversetzt sind,
- Figur 3: die erste Ausführungsform mit einer entfernten Abziehkappe,
- Figuren 4a, 4b: die erste Ausführungsform, wobei ein Primen freigegeben ist,
- Figuren 5a, 5b: die erste Ausführungsform nach dem Primen,
- Figuren 6a, 6b: die erste Ausführungsform während einer Dosiseinstellung,
- Figuren 7a, 7b: die erste Ausführungsform in einem Zustand, in der die Nadel über das distale Ende des Autoinjektors hervortritt und der Autoinjektor ausgelöst ist,
- Figuren 8a, 8b: die erste Ausführungsform während der Erzeugung eines Klickgeräuschs, welches den Beginn der Injektion signalisiert,
- Figuren 9a, 9b: die erste Ausführungsform in einem Zustand, in dem das Produkt ausgeschüttet ist,
- Figuren 10a, 10b: die erste Ausführungsform während der Erzeugung eines Klickgeräuschs, welches das Ende der Injektion signalisiert,
- Figuren 11a, 11b,c: die erste Ausführungsform in einem Zustand, in dem die Nadelschutzhülse die Nadel abdeckt und gegen Zurückschieben gesperrt ist,
- Figur 12: eine Explosionsdarstellung eines Autoinjektors gemäss einer zweiten Ausführungsform,
- Figur 13a, 13b: die zweite Ausführungsform in einem Auslieferungszustand, wobei die Figuren 13a und 13b durch die Längsachse der Vorrichtung verlaufende Schnittansichten sind, wobei die Schnittansichten um die Längsachse winkelversetzt sind,
- Figuren 14a, 14b: die zweite Ausführungsform mit einer entfernten Abziehkappe,
- Figur 15: die zweite Ausführungsform in einem Zustand, in dem ein Primen freigegeben ist,
- Figur 16: die zweite Ausführungsform, wobei ein Einstechen der Nadel freigegebenen ist,
- Figur 17: die zweite Ausführungsform in einem Zustand, in dem das Primen durchgeführt wurde,
- Figur 18: die zweite Ausführungsform in einem Zustand, in dem eine Dosisauswahl durchgeführt wurde,
- Figuren 19a, 19b: die zweite Ausführungsform in einem Zustand, in dem eine Nadel über das distale Ende des Autoinjektors hervortritt und eine Injektion freigegeben ist,
- Figur 20: die zweite Ausführungsform, bei der ein Klickgeräusch freigegeben wird, welches das Ende der Injektion signalisiert,
- Figur 21: die zweite Ausführungsform in einem Zustand, in dem das Klickgeräusch, welches das Ende der Injektion signalisiert, erzeugt wurde,
- Figur 22: die zweite Ausführungsform mit einer gegen Zurückschieben verriegelten Nadelschutzhülse in einer Nadelschutzposition,
- Figur 23: eine Explosionsdarstellung eines Autoinjektors gemäss einer dritten Ausführungsform,
- Figuren 24a-24c: die dritte Ausführungsform in einem Auslieferungszustand, wobei die Figuren 24a bis 24c durch die Längsachse der Vorrichtung verlaufende Schnittansichten sind, wobei die Schnittansichten um die Längsachse winkelversetzt sind,
- Figur 25: die dritte Ausführungsform mit einer entfernten Abziehkappe,
- Figuren 26a-26b: die dritte Ausführungsform während einer Dosiseinstellung,
- Figuren 27a-27b: die dritte Ausführungsform in einem ausgelösten Zustand,
- Figur 28: die dritte Ausführungsform, wobei ein Signal, welches den Beginn der Produktausschüttung signalisiert, erzeugt wird,
- Figur 29: die dritte Ausführungsform, wobei die Erzeugung eines Signals, welches das Ende der Produktausschüttung signalisiert, vorbereitet wird,
- Figuren 30a-30b: die dritte Ausführungsform nach erfolgter Ausschüttung der Produktdosis,
- Figuren 31a-31b: die dritte Ausführungsform, wobei das Signal, welches das Ende der Produktausschüttung signalisiert, erzeugt wird und
- Figuren 32a-32b: die dritte Ausführungsform mit einer gegen Zurückschieben verriegelten Nadelschutzhülse in einer Nadelschutzposition.

### Erste Ausführungsform

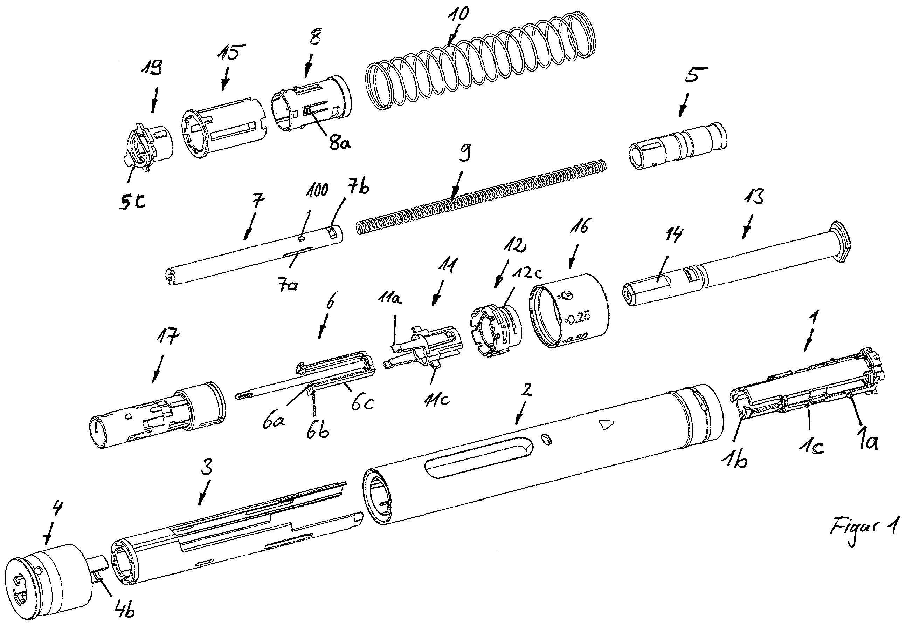

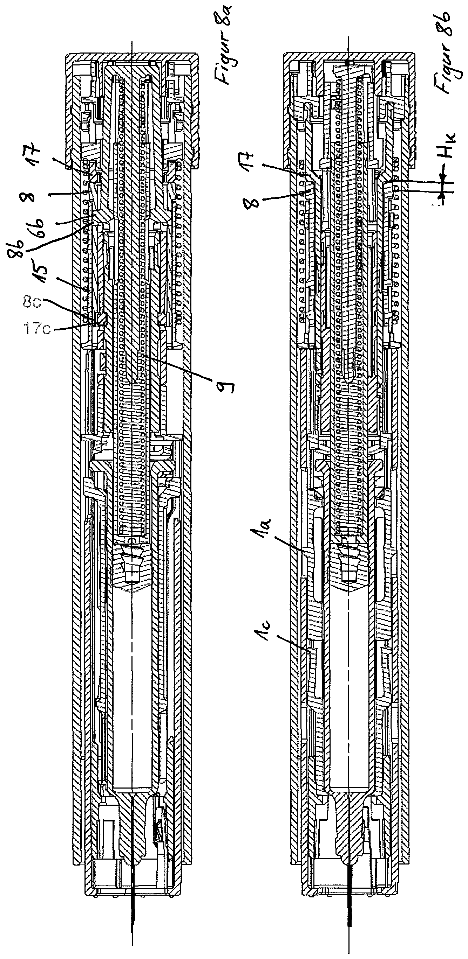

Bezugnehmend auf die Figuren 1 bis 11b werden nun die strukturellen Merkmale und die Funktion des Autoinjektors gemäss der ersten Ausführungsform beschrieben.

Der Autoinjektor weist ein hülsenförmiges, längliches Gehäuse 2 mit einer Längsachse L auf, welches an seinem proximalen Ende einen hülsenförmigen Gehäuseeinsatz 12 aufweist, der formschlüssig mit dem Gehäuse 2 dreh- und axialfest verbunden ist und am proximalen Ende des Gehäuses 2 angeordnet ist. Der hülsenförmige Gehäuseeinsatz 12 weist einen Rastschnapper 12c auf. Ein Dosiseinstellelement 16 weist mehrere, insbesondere mindestens zwei oder entsprechend der Anzahl einstellbarer Dosen über den Umfang verteilte Rastnuten 2f auf, wobei der Rastschnapper 12c in eine der Rastnuten 2f eingreift und bei einer Drehung des Dosiseinstellelements 16, wie zum Beispiel für eine Dosiseinstellung, aus der Rastnut 2f bewegt und in eine andere der Rastnuten 2f bewegt wird. Hierdurch werden quasistabile Rastpositionen für das Dosiseinstellelement 16 vorgegeben.

Das hülsenförmige Dosiseinstellelement 16 ist am proximalen Ende des Gehäuses 2 drehbar und axialfest mit dem Gehäuse 2 verbunden, insbesondere verschnappt, und bildet insbesondere das proximale Ende des Autoinjektors. Das Dosiseinstellelement 16 ist vom Verwender der Vorrichtung greifbar und relativ zu dem Gehäuse 2 per Muskelkraft des Verwenders für die Einstellung einer auszuschüttenden Produktdosis drehbar. Beispielsweise können mit der Vorrichtung mehrere variable Dosen in unterschiedlich kleinen Schritten eingestellt werden. Beispielsweise kann das Dosiseinstellelement 16 in Bezug auf das Gehäuse mindestens zwei oder, entsprechend der Anzahl der einstellbaren Dosen, zum Beispiel acht unterschiedliche quasistabile Drehpositionen einnehmen, wobei in einer ersten Drehposition eine erste Dosis, und in einer zweiten Drehposition eine zweite Dosis eingestellt ist usw.

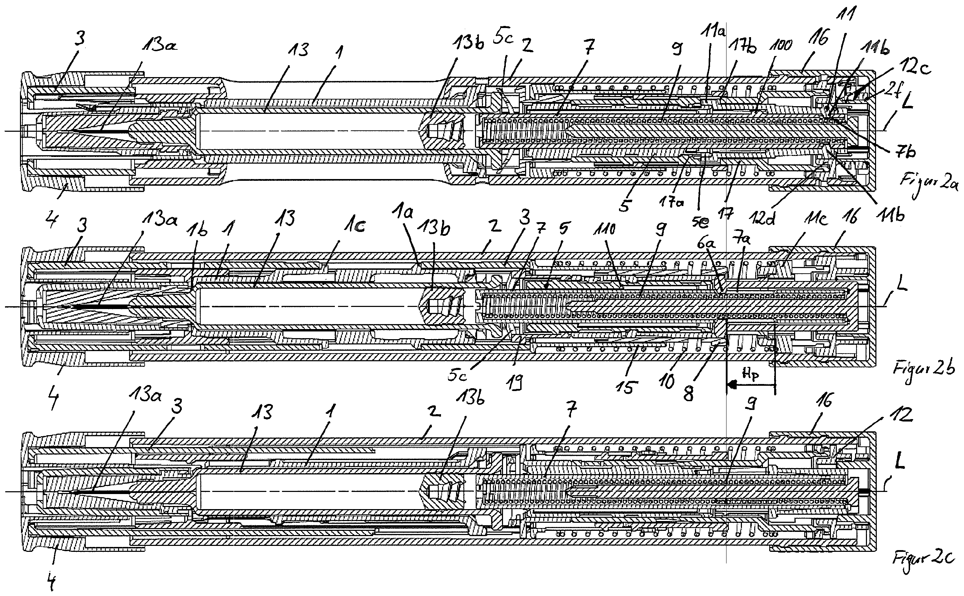

An dem distalen Ende des Autoinjektors ist in seinem Auslieferungszustand (Figuren 2a bis 2c) eine Abziehkappe 4 angeordnet, die vor der Verwendung des Autoinjektors abgezogen oder abgedreht, und entfernt wird.

In dem Gehäuse 2 ist ein Produktbehälter 13 in der Gestalt einer Spritze in Bezug auf das Gehäuse 2 - abgesehen von der Montage des Autoinjektors - entlang der Längsachse L unverschiebbar aufgenommen. Der Produktbehälter 13 weist einen hülsenförmigen Spritzkörper auf, der einen Kolben 13b, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel 13a auf, deren distales Ende von der Nadelspitze gebildet wird. Zwischen der Injektionsnadel 13a und dem Kolben 13b ist ein flüssiges Produkt, insbesondere Medikament, innerhalb des Spritzenkörpers angeordnet, wobei das Schieben des Kolbens 13b in eine Ausschüttrichtung, d. h. in die distale Richtung oder zu der Injektionsnadel 13a hin, das Produkt durch die hohle Injektionsnadel 13a aus dem Produktbehälter 13 ausschüttet. Der Spritzenkörper weist an seinem proximalen Ende einen sogenannten Fingerflansch auf, der radial nach aussen über den Umfang des zylindrischen Spritzenkörpers ragt.

Der Produktbehälter 13 ist in einem Produktbehälterhalter 1, der im Folgenden als Spritzenhalter 1 bezeichnet wird, so aufgenommen, dass er zumindest gegen eine Bewegung entlang der Längsachse L in die distale Richtung relativ zu dem Spritzenhalter 1 gesichert ist. Der Spritzenhalter 1 ist, wie zum Beispiel aus Figur 2a ersichtlich ist, mit dem Gehäuse 2 formschlüssig verbunden, insbesondere verrastet. Das Gehäuse 2 weist hierzu Ausnehmungen auf, in die Rastelemente, die hier am proximalen Ende des Spritzenhalters 1 gebildet werden, eingreifen. Der Spritzenhalter 1 weist mindestens eine nach innen ragende Schulter 1b auf, an der sich ein verjüngender Abschnitt des Produktbehälters 13, der distal des zylindrischen Spritzenkörperabschnitts, welcher den Kolben 13b führt, angeordnet ist, abstützt.

Der Autoinjektor weist eine in dem Gehäuse 2 angeordnete Dosierhülse 5 auf, die relativ zu dem Gehäuse 2 und einem Stössel 7 drehfest und axial bewegbar angeordnet ist. Die Dosierhülse 5 ist drehfest und axial verschiebbar mit einem Haltering 19 verbunden, der drehfest und vorzugsweise axialfest mit dem Gehäuse 2 verbunden, insbesondere verschnappt ist.

Um zu verhindern, dass der Produktbehälter 13 relativ zu dem Spritzenhalter 1 in die proximale Richtung verschiebbar ist, wird der Produktbehälter 13 an seinem proximalen Ende durch einen vom Haltering 19 gebildeten Haltefederabschnitt 5c in den Eingriff mit der Schulter 1b gedrückt. Durch den Haltefederabschnitt 5c können Längenunterschiede des Produktbehälters 13, die aufgrund von Herstellungstoleranzen entstehen können, ausgeglichen werden, wobei der feste Sitz des Produktbehälters 13 an der Schulter 1b sichergestellt ist.

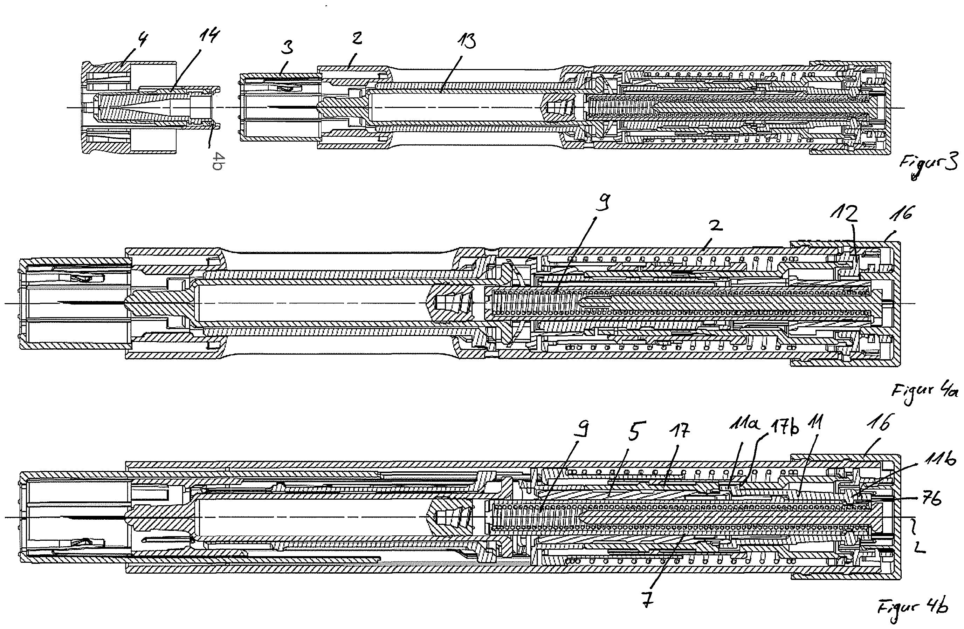

Der Produktbehälter 13 ist in Bezug auf das Gehäuse 2 so angeordnet, dass die Nadelspitze distal über das distale Ende des Gehäuses 2 steht. Im Ausgangs- oder Auslieferungszustand des Autoinjektors, d. h., wenn die Abziehkappe 4 an dem Autoinjektor angeordnet ist, wird die Nadel 13a von einer Nadelabdeckkappe 14, die in dem gezeigten Beispiel als sogenanntes und dem Fachmann bekanntes rigid needle shield, alternativ als soft needle shield, ausgebildet ist, abgedeckt, um die Nadel 13a vor Verschmutzung zu schützen bzw. die Nadel 13a und das Medikament steril zu halten. Das rigid needle shield 14 ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter 1b ist zwischen dem Spritzenkörper und dem proximalen Ende des rigid needle shield 14 angeordnet, insbesondere so, dass zwischen dem rigid needle shield 14 und der Schulter 1b ein - wenngleich auch geringer - Spalt entsteht, um zu verhindern, dass die Schulter 1b eine Kraft auf das rigid needle shield 14 ausübt, wodurch z. B. die Sterilität der Nadel 13a oder des flüssigen Produkts gefährdet werden könnte. Die Abziehkappe 4 ist mit dem Gehäuse 2 oder einer Nadelschutzhülse 3 lösbar verschnappt, wobei diese Verschnappung gelöst wird, wenn die Abziehkappe 4 von dem Gehäuse 2 oder der Nadelschutzhülse 3 entfernt wird. Die Abziehkappe 4 weist ferner insbesondere an einem flexiblen Arm mindestens einen Schnapper 4b auf, der in eine Lücke zwischen dem Spritzenkörper, insbesondere dessen sich verjüngenden Bereichs, und dem proximalen Ende des rigid needle shield 14 eingreift. Wenn die Abziehkappe 4 von dem Autoinjektor entfernt wird, hakt der Schnapper 4b in das proximale Ende des rigid needle shield 14 ein, wodurch das rigid needle shield 14 von dem Produktbehälter 13 gelöst und zusammen mit der Abdeckkappe 4 von dem Autoinjektor entfernt wird (Figur 3).

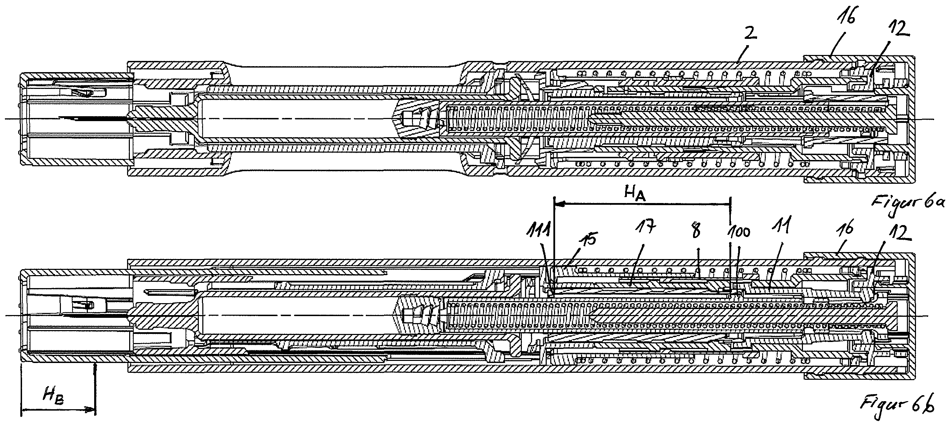

Der Autoinjektor weist eine Nadelschutzhülse 3 auf, die relativ zu dem Gehäuse 2 und entlang der Längsachse L um einen Betätigungshub H_{B} in die proximale Richtung in eine betätigte Position verschiebbar ist, um eine Produktausschüttung auszulösen. In der Ausgangsposition der Nadelschutzhülse 3, wie sie in den Figuren 6a, 6b gezeigt wird, wobei die Abziehkappe 4 abgenommen ist, steht das distale Ende der Nadelschutzhülse 3 distal über die Nadelspitze der Nadel 13a, so dass ein Zugriff auf die Nadelspitze zunächst verhindert wird. Durch Verschieben der Nadelschutzhülse 3 um den Betätigungshub H_{B} wird die Nadelschutzhülse 3 soweit in die proximale Richtung verschoben, dass die Nadel 13a aus dem distalen Ende der Nadelschutzhülse 3 hervortritt, insbesondere mit einer Länge hervortritt, welche der Injektionstiefe der Nadel in die Einstichstelle entspricht. Bevorzugt soll die Nadel 13a soweit über das distale Ende der Nadelschutzhülse 3 überstehen, dass eine subkutane Injektion erfolgen kann. Insbesondere kann das Gehäuse 2 einen Anschlag bilden, an dem die Nadelschutzhülse 3 in der betätigten Position anliegt.

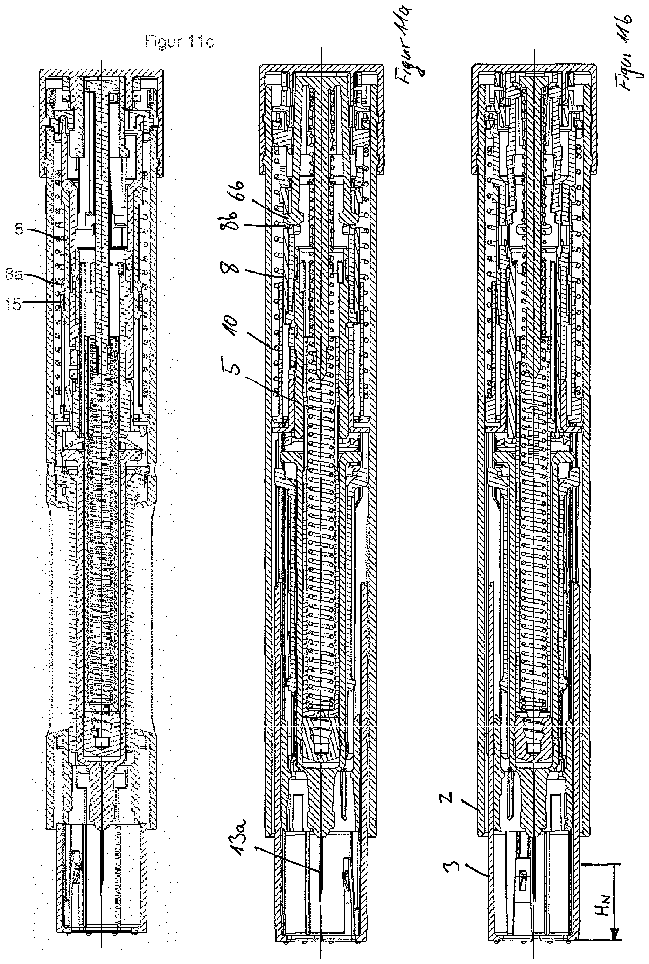

Nach der erfolgten Injektion kann die Nadelschutzhülse 3 relativ zu dem Gehäuse 2 aus der betätigten Position entlang der Längsachse L um einen Nadelschutzhub H_{N} in die distale Richtung in eine Nadelschutzposition (Figuren 11a, 11b) verschoben werden. In der Nadelschutzposition steht das distale Ende der Nadelschutzhülse 3 distal über die Nadelspitze, so dass ein Zugriff auf die Nadelspitze verhindert und eine Verletzungsgefahr verringert wird. Die Nadelschutzhülse 3 kann, wie weiter unten beschrieben wird, gegen erneutes Zurückschieben aus der Nadelschutzposition blockiert werden.

Der Spitzenhalter 1 weist eine Abragung 1a, die radial nach aussen weist, auf, wobei die Abragung 1a in eine schlitzförmige Ausnehmung der Nadelschutzhülse 3, die zwischen dem Gehäuse 2 und dem Spritzenhalter 1 angeordnet ist, eingreift. In der Ausgangsposition der Nadelschutzhülse 3 (Figuren 6a, 6b) und/oder in der Nadelschutzposition der Nadelschutzhülse 3 (Figuren 11a, 11b) liegt die Nadelschutzhülse 3, insbesondere das proximale Ende der schlitzförmigen Ausnehmung an der Abragung 1a an, wodurch eine Bewegung der Nadelschutzhülse 3 in die distale Richtung verhindert wird. In diese schlitzförmige Ausnehmung, alternativ in eine andere Ausnehmung der Nadelschutzhülse 3, kann eine Nocke 1c, die federnd an dem Spritzenhalter 1 angeordnet und von dem Spritzenhalter 1 gebildet ist, eingreifen. Die Nocke 1c ist so gestaltet, dass bei dem Versuch, die Nadelschutzhülse 3 aus der Ausgangsposition in die betätigte Position zu verschieben, die Nocke 1c die Verschiebung der Nadelschutzhülse 3 zunächst verhindert, wobei die Nocke 1c herausgedrückt wird, wenn die auf die Nadelschutzhülse 3 zum Zurückschieben aufgebrachte Kraft einen gewissen Schwellwert überschreitet, wodurch die Nadelschutzhülse 3 schlagartig in die betätigte Position zurückgeschoben wird. Die Nadel 13a kann hierdurch schlagartig in die Einstichstelle eingestochen werden. Zum Einstechen der Nadel 13a bzw. zum Verschieben der Nadelschutzhülse 3 in die betätigte Position wird das distale Ende der Nadelschutzhülse 3 an die Einstichstelle angesetzt, wobei das Gehäuse 2 dann in Richtung Einstichstelle gedrückt wird, wobei wenn die Andrückkraft den oben genannten Schwellwert überschreitet, das Gehäuse 2 schlagartig zu der Einstichstelle hin und die Nadelschutzhülse 3 relativ zu dem Gehäuse 2 um den Betätigungshub H_{B} in die betätigte Position verschoben wird.

Der Autoinjektor weist einen hülsenförmigen Stössel 7 auf, welcher an seinem distalen Ende eine nach innen ragende Schulter bildet, an der sich eine erste Feder 9, die auch als Ausschüttfeder bezeichnet werden kann, abstützt. Die erste Feder 9 ist innerhalb des hülsenförmigen Stössels 7 angeordnet. Die erste Feder 9 ist eine als Druckfeder wirkende Wendelfeder, die im Ausgangs- oder Auslieferungszustand des Autoinjektors mit so viel Energie vorgespannt ist, dass sie das in dem Produktbehälter 13 enthaltene Produkt insbesondere vollständig durch Verschieben des Stössels 7 um eine Ausschütthub H_{A} aus dem Produktbehälter 13 ausschütten kann. Im Auslieferungszustand der Vorrichtung besteht zwischen dem Kolben 13b und dem distalen Ende des Stössels 7 ein Abstand, so dass der Stössel 7 erst während der Ausführung eines Priminghubs H_{P} an den Kolben 13b anschlägt und diesen in die Ausschüttrichtung mitnimmt. Die erste Feder 9 stützt sich mit ihrem proximalen Ende an einem Halteelement 6 ab, welches in diesem Beispiel zwei Arme 6c aufweist, wobei an jedem Arm 6c ein erstes Eingriffselement 6a und ein zweites Eingriffselement 6b angeordnet ist. Das erste Eingriffselement 6a weist radial zu der Längsachse L hin, wobei das zweite Eingriffselement 6b radial von der Längsachse L weg weist. Das erste Eingriffselement 6a greift in eine erste Ausnehmung 7a, die von dem Stössel 7 gebildet wird, ein. Im Auslieferungszustand des Autoinjektors oder vor der Durchführung eines Primens weist das proximale Ende der ersten Ausnehmung 7a einen Abstand zu dem ersten Eingriffselement 6a auf, der dem Priminghub Hp entspricht (Figur 2b). Eine Sperrhülse 8, die entlang der Längsachse L verschiebbar ist, hält das erste Eingriffsglied 6a insbesondere mit ihrer Innenumfangsfläche in dem Eingriff mit der Ausnehmung 7a.

Ein hülsenförmiges Verschiebeglied 11 weist ein flexibles erstes Eingriffsglied 11a auf, welches in eine Ausnehmung 17b eines hülsenförmigen Mechanikhalters 17 eingreift, wodurch das Verschiebeglied 11 relativ dem Mechanikhalter 17 axialfest ist, solange der Eingriff besteht. Der Mechanikhalter 17 ist relativ zum Gehäuse 2 axialfest und drehbar, insbesondere axialfest und drehbar mit dem hülsenförmigen Gehäuseeinsatz 12 verschnappt. Das Verschiebeglied 11 weist ein zweites Eingriffsglied 11b auf, welches in eine zweite Ausnehmung 7b des Stössels 7 eingreift, wodurch der Stössel 7 von dem zweiten Eingriffsglied 17b gegen eine Bewegung in die distale Richtung blockiert ist. Der hülsenförmige Gehäuseeinsatz 12 weist eine Haltefläche 12d auf, an welcher das zweite Eingriffsglied 11b anliegt und im Eingriff mit der zweiten Ausnehmung 7b gehalten wird.

Der Stössel 7 weist an seinem Aussenumfang einen Dosisauswahlanschlag 100 in der Gestalt einer Abragung auf. Die Dosierhülse 5 weist über ihren Umfang verteilt mehrere, nämlich mindestens einen ersten Dosieranschlag 110 (Figur 2b) und zweiten Dosieranschlag 111 (Figur 6b) auf, die zueinander um die Längsachse L winkelversetzt und entlang der Längsachse L auf verschiedenen axialen Positionen angeordnet sind. Die Dosierhülse 5 weist insbesondere an ihrem Aussenumfang einen Stoppanschlag 5e auf, der entlang der Längsachse L einem Stoppgegenanschlag 17a, der von dem Mechanikhalter 17 gebildet ist, beabstandet gegenüberliegt (Figur 2a).

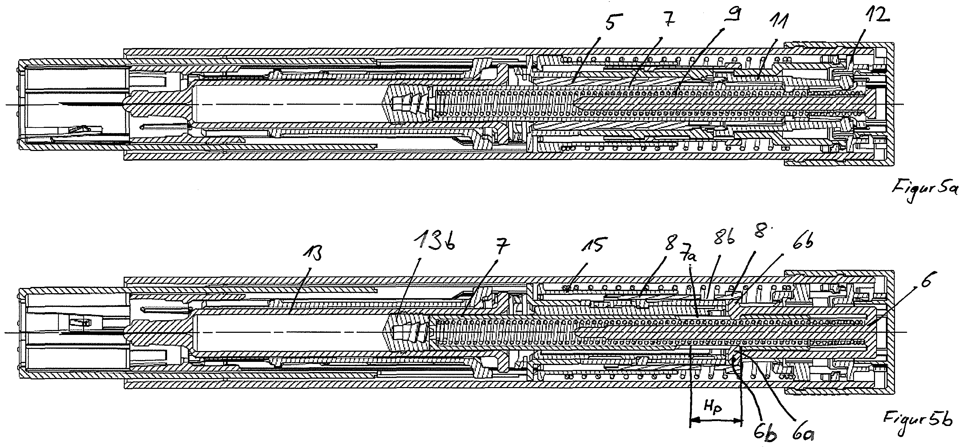

Zum Verabreichen des Medikaments wird die Abziehkappe 4 zusammen mit dem rigid needle shield 14 von dem Autoinjektor entfernt (Figur 3). Anschliessend wird der Autoinjektor geprimt (Figuren 4a, 4b). Hierfür wird das Dosiseinstellelement 16 relativ zu dem Gehäuse 2 gedreht. Der Stössel 7 ist insbesondere über das Haltelement 6 drehfest mit dem Dosiseinstellelement 16 verbunden. Das Verschiebeglied 11 ist drehfest mit dem Stössel 7 und dem Mechanikhalter 17 verbunden. Der Mechanikhalter 17 ist wiederum drehfest mit der Sperrhülse 8 verbunden, wobei die Sperrhülse 8 drehfest mit einer Schalthülse 15 verbunden ist. Beim Drehen des Dosiseinstellelements 16 wird insbesondere das Verschiebeglied 11 mitgedreht, wobei das zweite Eingriffsglied 11b aus dem Wirkeingriff mit der Haltefläche 12d des in Bezug auf das Gehäuse 2 drehfesten Gehäuseeinsatzes 12 gedreht, wobei in einer Position, in der das zweite Eingriffsglied 11b und die Haltefläche 12d um die Längsachse L winkelversetzt zueinander sind, insbesondere in der ersten oder zweiten Drehposition, das Eingriffsglied 11b aus dem Eingriff mit der zweiten Ausnehmung 7b durch die Wirkung der vorgespannten Ausschüttfeder 9 gedrückt wird, insbesondere mit einer Bewegung quer zur Längsachse L (Figur 4b). Die vorgespannte Ausschüttfeder 9 verschiebt den Stössel 7 um den Priminghub H_{P} in die distale Richtung, bis das proximale Ende der ersten Ausnehmung 7a an dem ersten Eingriffselement 6a anschlägt, wodurch die Bewegung des Stössels 7 in die distale Richtung blockiert wird. Während des Priminghubs H_{P} nimmt der Stössel 7 den Kolben 13b mit, wodurch die Spritze 13 geprimt wird (Figur 5b).

Nun kann die Dosiseinstellung erfolgen (Figuren 6a, 6b). Hierzu wird das Dosiseinstellelement 16 in eine der mehreren Drehpositionen, wie zum Beispiel die erste oder zweite Drehposition in Bezug auf das Gehäuse 2 gedreht. Der Stössel 7, das Halteelement 6, das Verschiebeglied 11, der Mechanikhalter 17, die Sperrhülse 8, die Schalthülse 15 und eine zweite Feder 10 drehen sich mit dem Dosiseinstellelement 16 mit. Insbesondere wird durch die Drehung des Dosiseinstellelements 16 der Dosisauswahlanschlag 100 relativ zu dem ersten und zweiten Dosieranschlag 110, 111 verdreht. In der ersten Drehposition des Dosiseinstellelements 16 sind der Dosisauswahlanschlag 100 und der erste Dosieranschlag 110 in einer Flucht entlang der Längsachse L angeordnet. In der zweiten Drehposition des Dosiseinstellelements 16 ist der Dosisauswahlanschlag 100 um die Längsachse L winkelversetzt zu dem ersten Dosieranschlag 110 und in einer Flucht mit dem zweiten Dosieranschlag 111 angeordnet (Figur 6b).

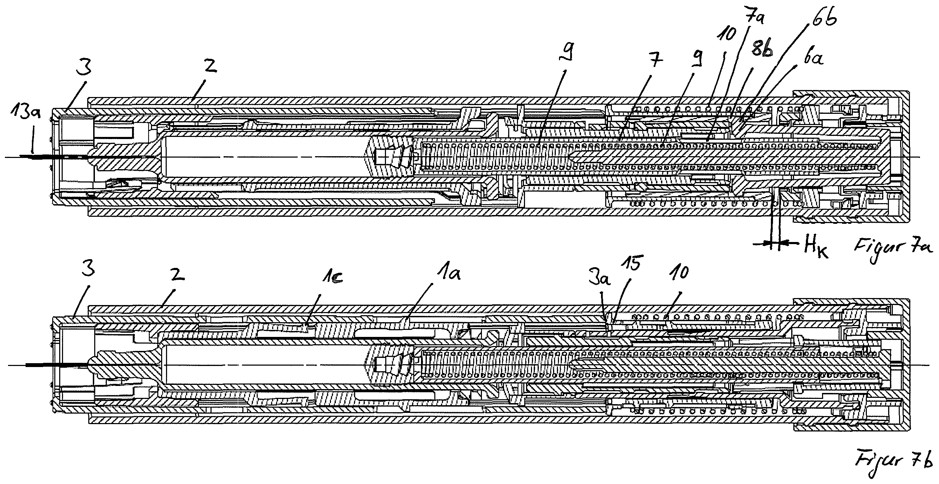

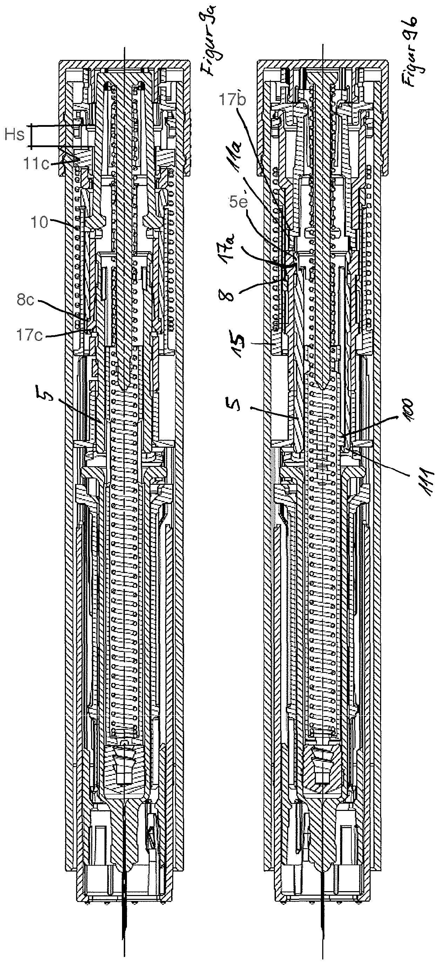

Zum Auslösen der Verabreichung der eingestellten Produktdosis, d. h. zum Lösen des Eingriffs des ersten Eingriffsglieds 6a aus der Ausnehmung 7a des Stössels 7 wird die Nadelschutzhülse 3 um den Betätigungshub H_{B} in die proximale Richtung in das Gehäuse 2 verschoben. Hierdurch nimmt das proximale Ende 3a der Nadelschutzhülse 3 die Schalthülse 15 mit, wobei die zweite Feder 10, insbesondere Nadelschutzfeder 10, gespannt wird. Die Schalthülse 15 schlägt während ihrer Bewegung in die proximale Richtung an der Sperrhülse 8 an und nimmt die Sperrhülse 8 ebenfalls mit. Durch die Bewegung der Sperrhülse 8 in die proximale Richtung wird eine erste Ausnehmung 8b der Sperrhülse 8 entlang der Längsachse L in etwa auf die Position des zweiten Eingriffsglieds 6b verschoben, wodurch die vorgespannte Ausschüttfeder 9 den Stössel 7 um einen Ausschütthub H_{A} in die distale Richtung verschiebt, wodurch das erste Eingriffsglied 6a aus dem Eingriff mit der Ausnehmung 7a und gleichzeitig das zweite Eingriffsglied 6b in die Ausnehmung 8b verschoben werden, insbesondere mit einer Bewegung quer zur Längsachse L (Figur 7a). Durch die Bewegung der Sperrhülse 8 in die proximale Richtung wird eine zweite Ausnehmung 8c der Sperrhülse 8 entlang der Längsachse L in etwa auf die Position eines Verriegelungsglieds 17c des Mechanikhalters 17 verschoben. Der Dosisauswahlanschlag 100 wird zu dem zweiten Dosieranschlag 111 hin und an dem ersten Dosieranschlag 110 vorbeibewegt, bis der Dosisauswahlanschlag 100 an dem zweiten Dosieranschlag 111 anstösst und die Dosierhülse 5 zumindest ein Stück weit, nämlich um den Abstand zwischen dem Stoppanschlag 5e und dem Stoppgegenanschlag 17a mitnimmt, bis der Stoppanschlag 5e an dem Stoppgegenanschlag 17a anstösst, wodurch die Bewegung der Dosierhülse 5 in die distale Richtung somit auch die Bewegung des Stössels 7 in die distale Richtung blockiert werden. Durch die Bewegung der Dosierhülse 5 in die distale Richtung wird sie aus der Position verschoben, in der sie verhindert, dass das erste Eingriffselement 11a aus der Ausnehmung 17b bewegbar ist (Figur 9b). Durch die Bewegung der Dosierhülse 5 in die distale Richtung drückt die Dosierhülse 5 das von dem Mechanikhalter 17 gebildete Verriegelungsglied 17c in die zweite Ausnehmung 8c der Sperrhülse 8, insbesondere mit einer Bewegung quer zur Längsachse L (Figur 7a), wodurch eine Bewegung der Sperrhülse 8 in die distale Richtung blockiert wird.

Wenn das zweite Eingriffselement 6b in den Eingriff mit der Ausnehmung 8b der Sperrhülse 8 gerät, kann das proximale Ende der Ausschüttfeder 9 das Halteelement 6 in die proximale Richtung beschleunigen, wobei das Eingriffselement 6b die Sperrhülse 8 in die proximale Richtung mitbeschleunigt, wobei die Sperrhülse 8 nach Ausführung eines Signalhubs H_{K} an dem Mechanikhalter 17 anschlägt und hierdurch ein taktiles und/oder akustisches Signal erzeugt (Figuren 7a bis 8b), das den Beginn der Produktausschüttung signalisiert. Zudem wird mittels des zweiten Eingriffselements 6b welcher sich im Eingriff mit der Ausnehmung 8b der Sperrhülse 8 befindet, die Bewegung der Sperrhülse 8 in die distale und proximale Richtung blockiert.

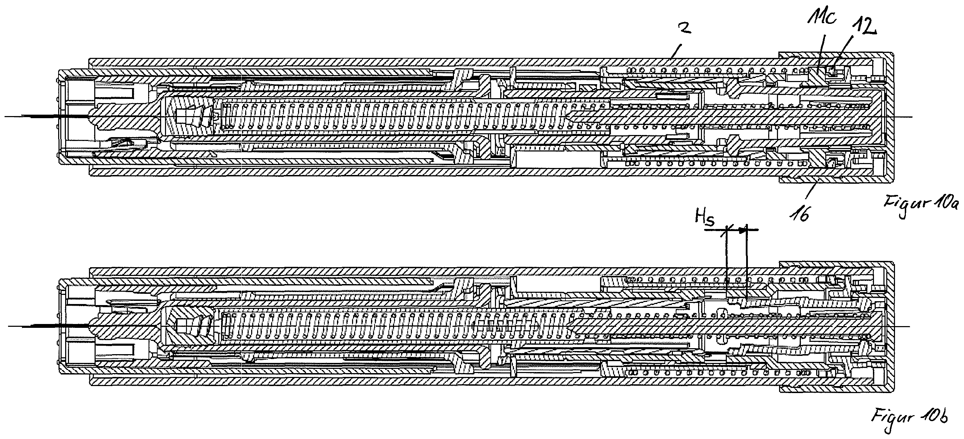

Da - wie aus den Figuren 9a, 9b ersichtlich ist - die Blockierung des ersten Eingriffsglieds 11a durch die Dosierhülse 5 am Ende des Ausschütthubs H_{A} weggefallen ist, kann die gespannte zweite Feder 10, die sich mit ihrem proximalen Ende an einer Abragung 11c des Verschiebeglieds 11 abstützt, das Verschiebeglied 11 in die proximale Richtung beschleunigen, wobei sich das erste Eingriffsglied 11a aus dem Eingriff mit der ersten Ausnehmung 17b löst. Das Verschiebeglied 11 wird um einen Signalhub H_{S} in die proximale Richtung beschleunigt bis es an dem hülsenförmigen Gehäuseeinsatz 12 anschlägt und ein akustisches und/oder taktiles Signal erzeugt, welches das Ende der Produktausschüttung anzeigt. Die Bewegung des Verschiebeglieds 11 in die proximale Richtung bewirkt ferner, dass das Verschiebeglied 11 insbesondere die Abragung 11c in einen formschlüssig verdrehfesten Eingriff mit dem Gehäuse 2 oder dem hülsenförmigen Gehäuseeinsatz 12 bewegt wird, wodurch eine Drehung des Verschiebeglieds 11 relativ zu dem Gehäuse 2 um die Längsachse L nach erfolgter Produktausschüttung verhindert wird. Da das Dosiseinstellelement 16 drehfest mit dem Verschiebeglied 11 verbunden ist, wird eine Drehung des Dosiseinstellelements 16 relativ zu dem Gehäuse 2 blockiert (Figur 10a).

Beim Abnehmen des Autoinjektors von der Einstichstelle, verschiebt die zweite Feder 10 die Schalthülse 15 relativ zu dem Gehäuse 2 und/oder der Sperrhülse 8 in die distale Richtung, wobei die Schalthülse 15 die Nadelschutzhülse 3 in die distale Richtung um den Nadelschutzhub H_{N} verschiebt. Durch den Eingriff des zweiten Eingriffselements 6b mit der Ausnehmung 8b der Sperrhülse 8, wird die Bewegung der Sperrhülse 8 in die distale und proximale Richtung gehindert, so dass die Schalthülse 15 sich relativ zu der Sperrhülse 8 bewegt und am Ende der Bewegung axialfest mit der Sperrhülse 8 verschnappt, insbesondere mittels eines in die Schalthülse 15 einrastenden Verriegelungsglieds 8a (Figur 11c) der Sperrhülse 8, so dass beim Versuch die Nadelschutzhülse 3 in die proximale Richtung zu verschieben, diese Bewegung blockiert wird. Die Sperrhülse 8 wird dabei gegen den axialfest mit dem Gehäuse 2 verbundenen Mechanikhalter 17 gedrückt, der die Bewegung der Sperrhülse 8 in die proximale Richtung blockiert.

### Zweite Ausführungsform

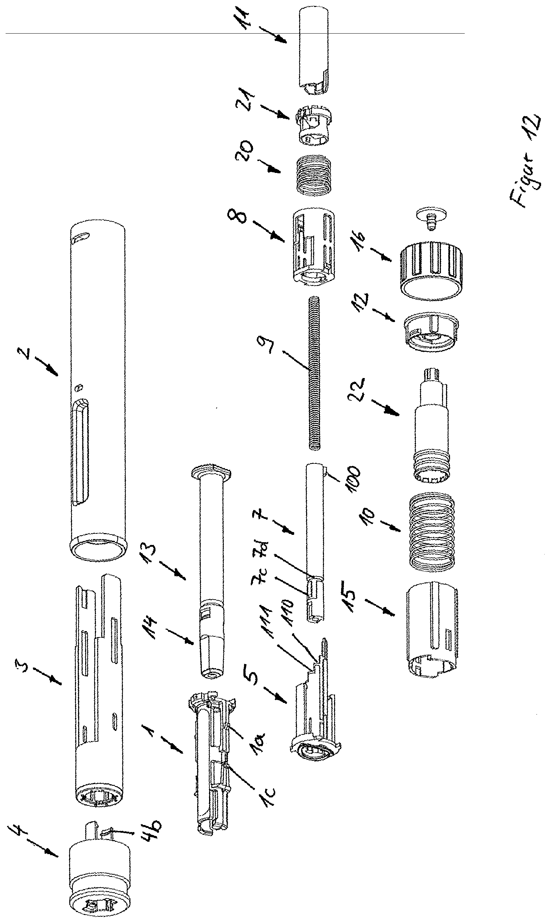

Bezugnehmend auf die Figuren 12 bis 22 werden nun die strukturellen Merkmale und die Funktion des Autoinjektors gemäss der zweiten Ausführungsform beschrieben.

Der Autoinjektor weist ein hülsenförmiges, längliches Gehäuse 2 mit einer Längsachse L auf, welches an seinem proximalen Ende einen hülsenförmigen Gehäuseeinsatz 12 aufweist, der formschlüssig mit dem Gehäuse 2 dreh- und axialfest verbunden ist und am proximalen Ende des Gehäuses 2 angeordnet ist. Der hülsenförmige Gehäuseeinsatz 12 weist einen Rastschnapper auf. Ein Dosiseinstellelement 16 weist mehrere, insbesondere mindestens zwei oder entsprechend der Anzahl einstellbarer Dosen über den Umfang verteilte Rastnuten auf, wobei der Rastschnapper in eine der Rastnuten eingreift und bei einer Drehung des Dosiseinstellelements 16, wie zum Beispiel für eine Dosiseinstellung, aus der Rastnut bewegt und in eine andere der Rastnuten bewegt wird. Hierdurch werden quasistabile Rastpositionen für das Dosiseinstellelement 16 vorgegeben.

Das hülsenförmige Dosiseinstellelement 16 ist am proximalen Ende des Gehäuses 2 drehbar und axialfest mit dem Gehäuse 2 verbunden, insbesondere verschnappt, und bildet insbesondere das proximale Ende des Autoinjektors. Das Dosiseinstellelement 16 ist vom Verwender der Vorrichtung greifbar und relativ zu dem Gehäuse 2 per Muskelkraft des Verwenders für die Einstellung einer auszuschüttenden Produktdosis drehbar. Beispielsweise können mit der Vorrichtung mehrere variable Dosen in verschiedenen ml-Schritten eingestellt werden. Beispielsweise kann das Dosiseinstellelement 16 in Bezug auf das Gehäuse mindestens zwei oder, entsprechend der Anzahl der einstellbaren Dosen, zum Beispiel acht unterschiedliche quasistabile Drehpositionen einnehmen, wobei in einer ersten Drehposition eine erste Dosis, und in einer zweiten Drehposition eine zweite Dosis eingestellt ist usw.

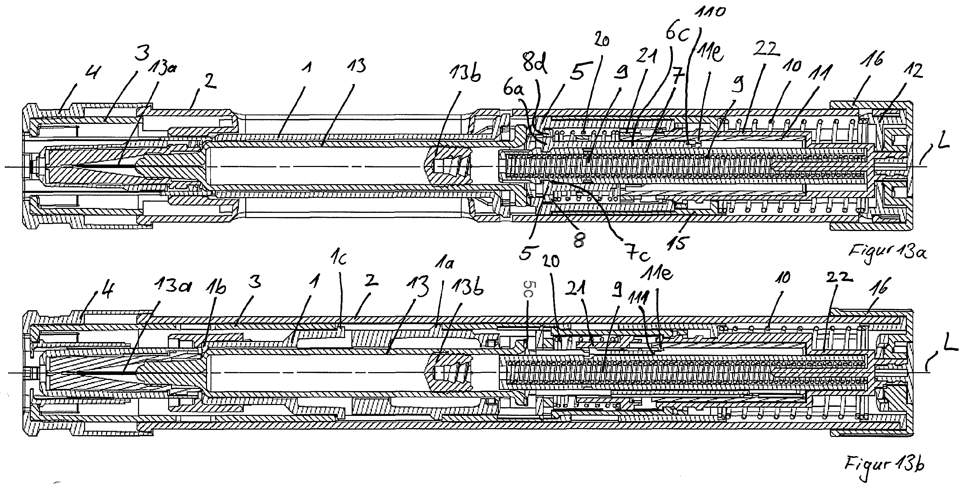

An dem distalen Ende des Autoinjektors ist in seinem Auslieferungszustand (Figuren 13a, 13b) eine Abziehkappe 4 angeordnet, die vor der Verwendung des Autoinjektors abgezogen oder abgedreht, und entfernt wird.

In dem Gehäuse 2 ist ein Produktbehälter 13 in der Gestalt einer Spritze in Bezug auf das Gehäuse 2 - abgesehen von der Montage des Autoinjektors - entlang der Längsachse L unverschiebbar aufgenommen. Der Produktbehälter 13 weist einen hülsenförmigen Spritzkörper auf, der einen Kolben 13b, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel 13a auf, deren distales Ende von der Nadelspitze gebildet wird. Zwischen der Injektionsnadel 13a und dem Kolben 13b ist ein flüssiges Produkt, insbesondere Medikament, innerhalb des Spritzenkörpers angeordnet, wobei das Schieben des Kolbens 13b in eine Ausschüttrichtung, d. h. in die distale Richtung oder zu der Injektionsnadel 13a hin, das Produkt durch die hohle Injektionsnadel 13a aus dem Produktbehälter 13 ausschüttet. Der Spritzenkörper weist an seinem proximalen Ende einen sogenannten Fingerflansch auf, der radial nach aussen über den Umfang des zylindrischen Spritzenkörpers ragt.

Der Produktbehälter 13 ist in einem Produktbehälterhalter 1, der im Folgenden als Spritzenhalter 1 bezeichnet wird, so aufgenommen, dass er zumindest gegen eine Bewegung entlang der Längsachse L in die distale Richtung relativ zu dem Spritzenhalter 1 gesichert ist. Der Spritzenhalter 1 ist, wie zum Beispiel aus Figur 13a ersichtlich ist, mit dem Gehäuse 2 formschlüssig verbunden, insbesondere verrastet. Das Gehäuse 2 weist hierzu Ausnehmungen auf, in die Rastelemente, die hier am proximalen Ende des Spritzenhalters 1 gebildet werden, eingreifen. Der Spritzenhalter 1 weist mindestens eine nach innen ragende Schulter 1b auf, an der sich ein verjüngender Abschnitt des Produktbehälters 13, der distal des zylindrischen Spritzenkörperabschnitts, welcher den Kolben 13b führt, angeordnet ist, abstützt.

Der Autoinjektor weist eine in dem Gehäuse 2 angeordnete Dosierhülse 5 auf, die relativ zu dem Gehäuse 2 und einem Stössel 7 drehfest und axial bewegbar angeordnet ist. Die Dosierhülse 5 greift drehfest und axial verschiebbar in das Gehäuse 2 ein. Die Dosierhülse 5 weist ein Eingriffselement 6a auf, welches an einem Arm 6c so von der Dosierhülse 5 insbesondere monolithisch gebildet ist, dass es quer zur Längsachse L flexibel auslenkbar ist. Das Eingriffselement 6a greift in einen Stössel 7 ein und blockiert den Stössel 7 gegen eine Bewegung in die distale Richtung. Auf den Stössel 7 wirkt eine vorgespannte erste Feder 9, welche über das Eingriffselement 6a die Dosierhülse 5 in die distale Richtung mit einer Kraft beaufschlagt bzw. drückt, um zu verhindern, dass der Produktbehälter 13 relativ zu dem Spritzenhalter 1 in die proximale Richtung verschiebbar ist. Der Produktbehälter 13 wird an seinem proximalen Ende durch einen von der Dosierhülse 5 gebildeten Haltefederabschnitt 5c in dem Eingriff mit der Schulter 1b gehalten.

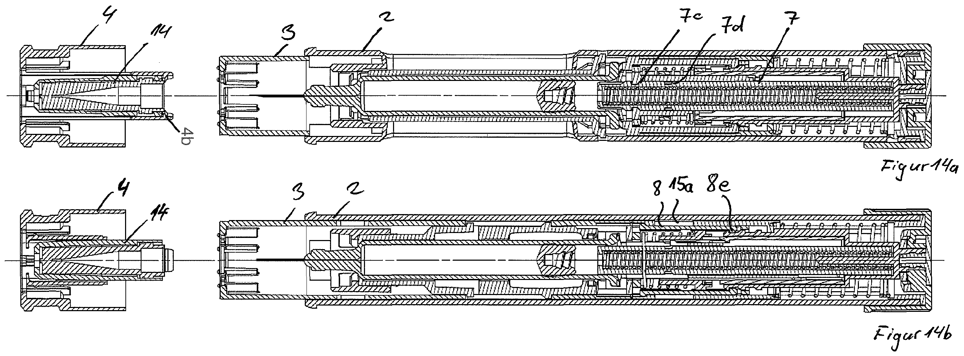

Der Produktbehälter 13 ist in Bezug auf das Gehäuse 2 so angeordnet, dass die Nadelspitze distal über das distale Ende des Gehäuses 2 steht. Im Ausgangs- oder Auslieferungszustand des Autoinjektors, d. h., wenn die Abziehkappe 4 an dem Autoinjektor angeordnet ist, wird die Nadel 13a von einer Nadelabdeckkappe 14, die in dem gezeigten Beispiel als sogenanntes und dem Fachmann bekanntes rigid needle shield, alternativ als soft needle shield, ausgebildet ist, abgedeckt, um die Nadel 13a vor Verschmutzung zu schützen bzw. die Nadel 13a und das Medikament steril zu halten. Das rigid needle shield 14 ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter 1b ist zwischen dem Spritzenkörper und dem proximalen Ende des rigid needle shield 14 angeordnet, insbesondere so, dass zwischen dem rigid needle shield 14 und der Schulter 1b ein - wenngleich auch geringer - Spalt entsteht, um zu verhindern, dass die Schulter 1b eine Kraft auf das rigid needle shield 14 ausübt, wodurch z. B. die Sterilität der Nadel 13a oder des flüssigen Produkts gefährdet werden könnte. Die Abziehkappe 4 ist mit dem Gehäuse 2 oder einer Nadelschutzhülse 3 lösbar verschnappt, wobei diese Verschnappung gelöst wird, wenn die Abziehkappe 4 von dem Gehäuse 2 oder der Nadelschutzhülse 3 entfernt wird. Die Abziehkappe 4 weist ferner insbesondere an einem flexiblen Arm mindestens einen Schnapper 4b auf, der in eine Lücke zwischen dem Spritzenkörper, insbesondere dessen sich verjüngenden Bereichs, und dem proximalen Ende des rigid needle shield 14 eingreift. Wenn die Abziehkappe 4 von dem Autoinjektor entfernt wird, hakt der Schnapper 4b in das proximale Ende des rigid needle shield 14 ein, wodurch das rigid needle shield 14 von dem Produktbehälter 13 gelöst und zusammen mit der Abdeckkappe 4 von dem Autoinjektor entfernt wird (Figuren 14a, 14b).

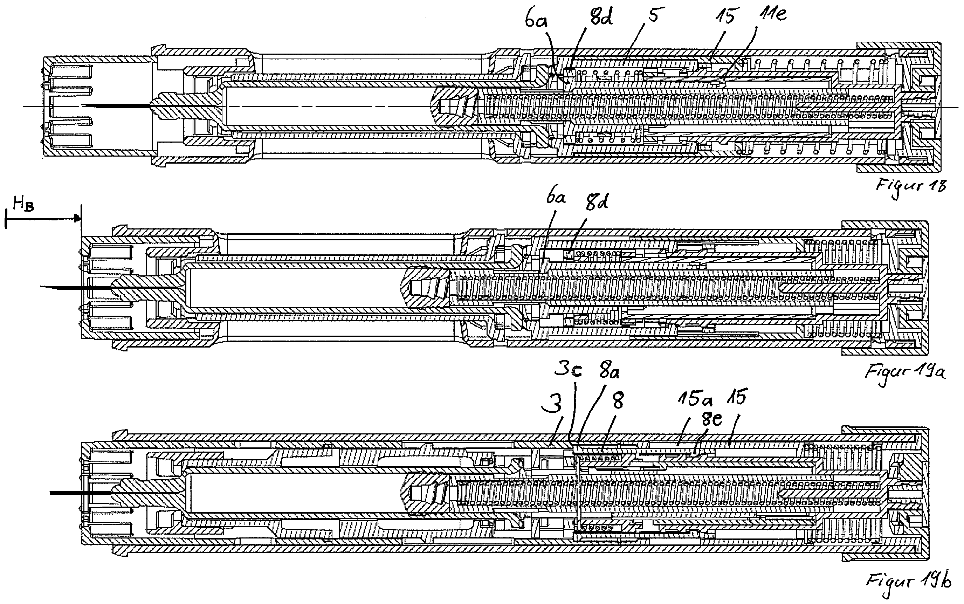

Der Autoinjektor weist eine Nadelschutzhülse 3 auf, die relativ zu dem Gehäuse 2 und entlang der Längsachse L um einen Betätigungshub H_{B} (Figur 19a) in die proximale Richtung in eine betätigte Position verschiebbar ist, um eine Produktausschüttung auszulösen. In der Ausgangsposition der Nadelschutzhülse 3, wie sie in den Figuren 14a bis 18b gezeigt wird, wobei die Abziehkappe 4 abgenommen ist, steht das distale Ende der Nadelschutzhülse 3 distal über die Nadelspitze der Nadel 13a, so dass ein Zugriff auf die Nadelspitze zunächst verhindert wird. Durch Verschieben der Nadelschutzhülse 3 um den Betätigungshub H_{B} wird die Nadelschutzhülse 3 soweit in die proximale Richtung verschoben, dass die Nadel 13a aus dem distalen Ende der Nadelschutzhülse 3 hervortritt, insbesondere mit einer Länge hervortritt, welche der Injektionstiefe der Nadel in die Einstichstelle entspricht. Bevorzugt soll die Nadel 13a soweit über das distale Ende der Nadelschutzhülse 3 überstehen, dass eine subkutane Injektion erfolgen kann. Insbesondere kann das Gehäuse 2 einen Anschlag bilden, an dem die Nadelschutzhülse 3 in der betätigten Position anliegt.

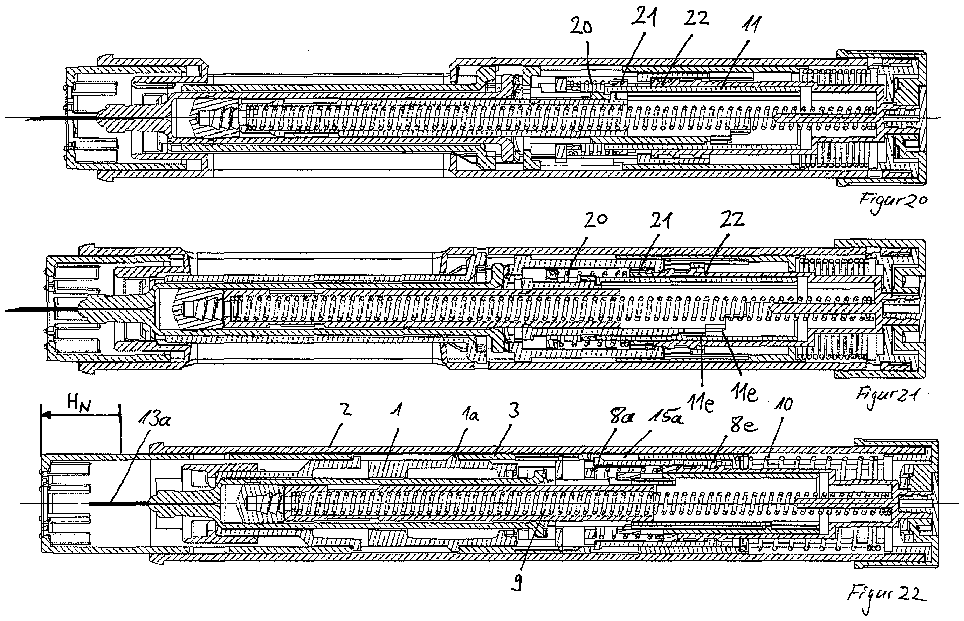

Nach der erfolgten Injektion kann die Nadelschutzhülse 3 relativ zu dem Gehäuse 2 aus der betätigten Position entlang der Längsachse L um einen Nadelschutzhub H_{N} in die distale Richtung in eine Nadelschutzposition (Figur 22) verschoben werden. In der Nadelschutzposition steht das distale Ende der Nadelschutzhülse 3 distal über die Nadelspitze, so dass ein Zugriff auf die Nadelspitze verhindert und eine Verletzungsgefahr verringert wird. Die Nadelschutzhülse 3 kann, wie weiter unten beschrieben wird, gegen erneutes Zurückschieben aus der Nadelschutzposition blockiert werden.

Der Spitzenhalter 1 weist eine Abragung 1a, die radial nach aussen weist, auf, wobei die Abragung 1a in eine schlitzförmige Ausnehmung der Nadelschutzhülse 3, die zwischen dem Gehäuse 2 und dem Spritzenhalter 1 angeordnet ist, eingreift. In der Ausgangsposition der Nadelschutzhülse 3 (Figuren 14a bis 18b) und/oder in der Nadelschutzposition der Nadelschutzhülse 3 (Figur 22) liegt die Nadelschutzhülse 3, insbesondere das proximale Ende der schlitzförmigen Ausnehmung an der Abragung 1a an, wodurch eine Bewegung der Nadelschutzhülse 3 in die distale Richtung verhindert wird. In diese schlitzförmige Ausnehmung, alternativ in eine andere Ausnehmung der Nadelschutzhülse 3, kann eine Nocke 1c, die federnd an dem Spritzenhalter 1 angeordnet und von dem Spritzenhalter 1 gebildet ist, eingreifen. Die Nocke 1c ist so gestaltet, dass bei dem Versuch, die Nadelschutzhülse 3 aus der Ausgangsposition in die betätigte Position zu verschieben, die Nocke 1c die Verschiebung der Nadelschutzhülse 3 zunächst verhindert, wobei die Nocke 1c herausgedrückt wird, wenn die auf die Nadelschutzhülse 3 zum Zurückschieben aufgebrachte Kraft einen gewissen Schwellwert überschreitet, wodurch die Nadelschutzhülse 3 schlagartig in die betätigte Position zurückgeschoben wird. Die Nadel 13a kann hierdurch schlagartig in die Einstichstelle eingestochen werden. Zum Einstechen der Nadel 13a bzw. zum Verschieben der Nadelschutzhülse 3 in die betätigte Position wird das distale Ende der Nadelschutzhülse 3 an die Einstichstelle angesetzt, wobei das Gehäuse 2 dann in Richtung Einstichstelle gedrückt wird, wobei wenn die Andrückkraft den oben genannten Schwellwert überschreitet, das Gehäuse 2 schlagartig zu der Einstichstelle hin und die Nadelschutzhülse 3 relativ zu dem Gehäuse 2 um den Betätigungshub H_{B} in die betätigte Position verschoben wird.

Der Autoinjektor weist einen hülsenförmigen Stössel 7 auf, welcher an seinem distalen Ende eine nach innen ragende Schulter bildet, an der sich eine erste Feder 9, die auch als Ausschüttfeder bezeichnet werden kann, abstützt. Die erste Feder 9 ist innerhalb des hülsenförmigen Stössels 7 angeordnet. Die erste Feder 9 ist eine als Druckfeder wirkende Wendelfeder, die im Ausgangs- oder Auslieferungszustand des Autoinjektors mit so viel Energie vorgespannt ist, dass sie das in dem Produktbehälter 13 enthaltene Produkt insbesondere vollständig durch Verschieben des Stössels 7 um eine Ausschütthub H_{A} aus dem Produktbehälter 13 ausschütten kann. Im Auslieferungszustand der Vorrichtung, d.h. vor einem Primen, besteht zwischen dem Kolben 13b und dem distalen Ende des Stössels 7 ein Abstand, so dass der Stössel 7 erst während der Ausführung eines Priminghubs H_{P} an dem Kolben 13b anschlägt und diesen in die Ausschüttrichtung mitnimmt. Die erste Feder 9 stützt sich mit ihrem proximalen Ende an einer Kupplungshülse 22 ab, welche relativ zu dem Gehäuse 2 drehbar und axialfest ist und mit dem Dosiseinstellelement 16 drehfest verbunden ist, insbesondere eingreift.

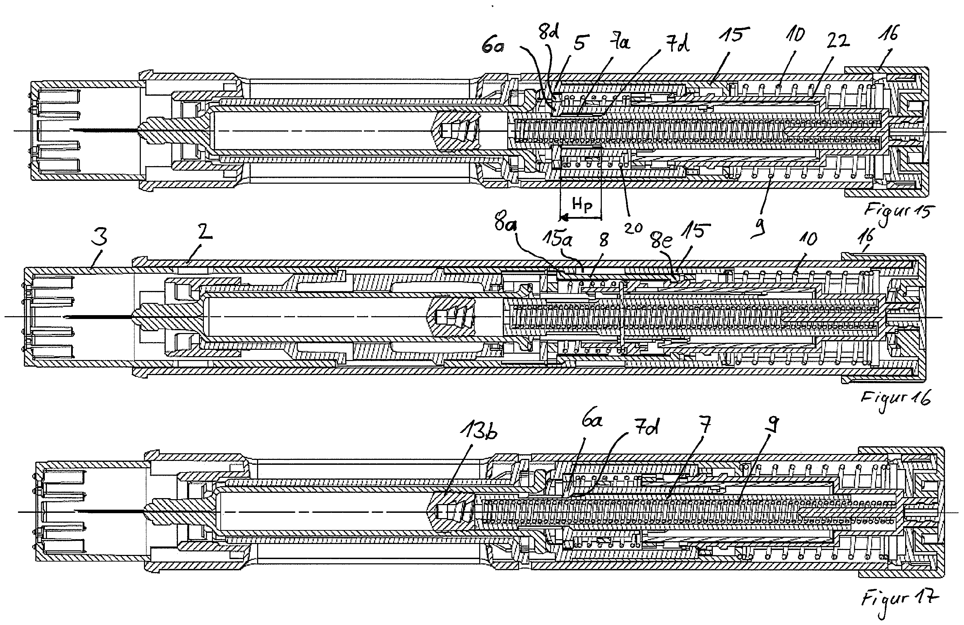

Das erste Eingriffselement 6a greift in eine Ausnehmung 7a, die von dem Stössel 7 gebildet wird, ein. Die Ausnehmung 7a weist eine erste Anschlagfläche 7c und eine zweite ringförmig über den Umfang des Stössels 7 umlaufende Anschlagfläche 7d auf, die entlang der Längsachse L zueinander versetzt angeordnet sind, insbesondere mit einem Abstand, der in etwa dem Priminghub H_{P} (Figur 15) entspricht. Die Ausnehmung 7a weist insbesondere einen sich entlang der Längsachse L erstreckenden Kanal auf, der die erste Anschlagfläche 7c mit der zweiten Anschlagfläche 7d verbindet. Im Auslieferungszustand des Autoinjektors oder vor der Durchführung eines Primens liegt das Eingriffselement 6a an der ersten Anschlagfläche 7c an, wodurch eine Bewegung des Stössels 7 in die distale Richtung blockiert ist. Eine entlang der Längsachse L verschiebbare Sperrhülse 8, insbesondere eine von ihr gebildete Haltefläche 8d, hält das erste Eingriffsglied 6a in dem Eingriff mit der Ausnehmung 7a, indem es eine Bewegung des ersten Eingriffsglieds 6a quer zur Längsachse L blockiert. Die Sperrhülse 8, insbesondere deren Haltefläche 8d, ist aus dieser Position in die proximale Richtung in eine Position verschiebbar, in der die Sperrhülse 8, insbesondere deren Haltefläche 8d, das erste Eingriffsglied 6a für eine Bewegung quer zur Längsachse L freigibt.

Mittels Drehen des Dosiseinstellelements 16 ist der Stössel 7 relativ zu der Dosierhülse 5 drehbar, wodurch die erste Anschlagfläche 7c aus der Anlage mit dem Eingriffselement 6a drehbar ist, wodurch der Stössel 7 mittels der vorgespannten ersten Feder 9 um den Priminghub Hp in die distale Richtung verschiebbar ist, wobei das Eingriffsglied 6a während des Priminghubs H_{P} in der Ausnehmung 7a gehalten wird, wobei am Ende des Priminghubs Hp das Eingriffselement 6a an der zweiten Anschlagfläche 7d anliegt, wodurch die Bewegung des Stössels 7 in die distale Richtung blockiert wird, insbesondere so lange blockiert wird, bis eine Produktdosis eingestellt und deren Ausschüttung gestartet wird.

Für die Produktausschüttung ist die Sperrhülse 8 in die proximale Richtung verschiebbar, wodurch ihre Haltefläche 8d das Eingriffselement 6a freigibt, aus der Ausnehmung 7a bzw. dem Eingriff mit der zweiten Anschlagfläche 7d bewegt zu werden, wodurch der Stössel 7 von der ersten Feder 9 in die distale Richtung verschiebbar bzw. die Verschiebung freigegeben ist.

Der Stössel 7 weist an seinem Aussenumfang einen Dosisauswahlanschlag 100 in der Gestalt einer Abragung auf. Die Dosierhülse 5 weist über ihren Umfang verteilt mehrere, nämlich mindestens einen ersten Dosieranschlag 110 (Figur 12) und zweiten Dosieranschlag 111 (Figur 12) auf, die zueinander um die Längsachse L winkelversetzt und entlang der Längsachse L auf verschiedenen axialen Positionen angeordnet sind.

Der Autoinjektor weist ein hülsenförmiges Verschiebeglied 11 auf, welches drehfest und axial verschiebbar an der Dosierhülse 5 gelagert ist. Das Verschiebeglied 11 weist an seinem Innenumfang mehrere Mitnahmeanschläge 11e auf, wobei jedem Dosieranschlag 110, 111 einer dieser Mitnahmeanschläge 11e zugeordnet ist, wobei der jeweilige Mitnahmeanschlag 11e in Bezug auf den Dosieranschlag 110, 111, dem er zugeordnet ist, in proximaler Richtung vorgelagert und so positioniert ist, dass er von dem Dosisauswahlelement 100 mitgenommen wird, wenn sein Dosieranschlag 110, 111 in einer Flucht mit dem Dosisauswahlelement 100 ist und das Dosisauswahlelement 100 zu dem Dosieranschlag 110, 111 hin bewegt wird. Hierdurch wird das Verschiebeglied 11 in die distale Richtung verschoben, insbesondere gegen Ende des Ausschütthubs H_{A}.

Der Autoinjektor weist einen um die Längsachse L drehbaren Ring 21 auf, welcher die Dosierhülse 5 umgibt. Der Ring 21 ist aus einer ersten Position, in welcher er relativ zu der Dosierhülse 5 drehbar und zumindest in die proximale Richtung axialfest ist, in eine zweite Position bewegbar, in welcher er relativ zu der Dosierhülse 5 drehfest ist. Der Ring 21 weist eine Abragung auf, welche an einer Schulter der Dosierhülse 5 anliegt, wobei die Schulter die Bewegung des Rings 21 in die proximale Richtung blockiert und eine Drehung des Rings 21 zulässt. Der Ring 21 weist eine sich entlang der Längsachse L erstreckende Nut auf, in welche die Abragung des Rings 21 mit einer Dreh-Axial-Bewegung bewegbar ist und welche die Abragung des Rings 21 beidseitig einfasst, so dass eine Drehung des Rings 21 relativ zu der Dosierhülse 5 blockiert ist.

Der Ring 21 wird mittels einer dritten vorgespannten Feder 20, die sich mit ihrem proximalen Ende am Ring 21 und mit ihrem distalen Ende an der Dosierhülse 5 abstützt, mit einer in die distale Richtung wirkenden Kraft beaufschlagt. Wird der Ring 21 aus der ersten Position, d.h. aus dem axialfesten Eingriff mit der Schulter gedreht, drückt die dritte Feder 20 den Ring 21 in die proximale Richtung, wodurch die Abragung des Rings 21 in die Nut bewegt wird.

Das Verschiebeglied 11 weist eine Getriebefläche auf, welche an einer Getriebegegenfläche des Rings 21 abgleitet, wenn das Verschiebeglied 11 in die distale Richtung verschoben wird, insbesondere gegen Ende der Produktausschüttung, wodurch der Ring 21 aus der ersten Position gedreht und von der zweiten Feder 20 in die zweite Position translatorisch verschoben wird.

Der Ring 21 kann von der Feder 20 gegen die Kupplungshülse 22 beschleunigt oder bewegt werden, wobei beim Anstossen des Rings 21 an der Kupplungshülse 22 ein taktiles und/oder akustisches Signal erzeugt wird, welches das Ende der Ausschüttung der ausgewählten Dosis signalisiert.

Der Ring 21 kann von der Feder 20 in eine Position, insbesondere die zweite Position, verschoben werden, in der der Ring 21 drehfest in die Kupplungshülse 22 eingreift, wobei die Kupplungshülse 22 drehfest mit dem Dosiseinstellelement 16 gekoppelt ist, insbesondere in einem drehfesten Einriff mit dieser ist, wodurch die Drehung des Dosiseinstellelements 16 relativ zu dem Gehäuse 2 blockiert wird.

Die Kupplungshülse 22 ist über einen lösbaren Kupplungseingriff zumindest vor der Auslösung der Produktausschüttung drehfest mit dem Stössel 7 gekoppelt, d.h. wenn das Eingriffselement 6a an der ersten Anschlagfläche 7c oder/und der zweiten Anschlagfläche 7d anliegt. Dadurch kann der Stössel 7 für die Einstellung der auszuschüttenden Dosis mit dem Dosiseinstellelement 16 mitgedreht werden. Hierfür greifen das proximale Ende des Stössels 7, insbesondere die Abragung mit dem Dosisauswahlanschlag 100, und die Kupplungshülse 22 verdrehfest ineinander. Der Kupplungseingriff ist mittels einer Bewegung des Stössels 7 in die distale Richtung lösbar, d.h. insbesondere mittels des Ausschütthubs H_{A}, wobei der Stössel 7 aus dem Eingriff mit der Kupplungshülse 22 in die distale Richtung verschoben wird. Wenn der Kupplungseingriff gelöst ist, d.h. während der Ausschüttung der Dosis oder wenn der Dosisauswahlanschlag 100 an einem der Dosieranschläge 110, 111, nämlich dem ausgewählten Dosieranschlag, anliegt, sind das Dosiseinstellelement 16 und der Stössel 7 rotatorisch voneinander entkoppelt.

Zum Verabreichen des Medikaments wird die Abziehkappe 4 zusammen mit dem rigid needle shield 14 von dem Autoinjektor entfernt (Figuren 14a). Anschliessend werden der Autoinjektor geprimt (Figur 15) und die Nadelschutzhülse 3 für eine Bewegung in die proximale Richtung freigegeben (Figur 16). Die Nadelschutzhülse 3 ist in der Ausgangsposition gegen eine Verschiebung in die proximale Richtung gesperrt. Hierfür stösst die Nadelschutzhülse 3, insbesondere deren proximales Ende, an die Schalthülse 15 an, wobei die Schalthülse 15 durch einen Blockieranschlag der Kupplungshülse gegen eine Bewegung in die proximale Richtung blockiert ist.

Nach dem Entfernen der Abziehkappe 4, wird das Dosiseinstellelement 16 relativ zu dem Gehäuse 2 gedreht. Der Stössel 7 ist insbesondere über die Kupplungshülse 22 drehfest mit dem Dosiseinstellelement 16 verbunden.

Durch das Drehen des Dosiseinstellelements 16 wird insbesondere die Kupplungshülse22 mitgedreht, wobei die Kupplungshülse 22 relativ zu der Schalthülse 15 verdreht wird, so dass der Blockieranschlag der Kupplungshülse 22 aus dem Eingriff mit der Schalthülse 15 gedreht wird. Hierdurch kann die Schalthülse 15 an dem Blockieranschlag vorbei in die proximale Richtung bewegt werden.

Durch das Drehen des Dosiseinstellelements 16 wird insbesondere der Stössel 7 relativ zu der Dosierhülse 5 gedreht, wodurch das Eingriffselement 6a aus der Anlage mit der ersten Anschlagfläche 7c gedreht wird, so dass der Stössel 7 sich mittels der vorgespannten Feder 9 um dem Priminghub H_{P} in die distale Richtung bewegt bis die zweite Anschlagfläche 7d an dem Eingriffselement 6a anschlägt und die Bewegung des Stössels 7 blockiert (Figur 17).

Nun kann die Dosiseinstellung erfolgen (Figur 18). Hierzu wird das Dosiseinstellelement 16 in eine der mehreren Drehpositionen, wie zum Beispiel die erste oder zweite Drehposition in Bezug auf das Gehäuse 2 gedreht. Der Stössel 7, die Kupplungshülse 22 und die erste Feder 9 drehen sich mit dem Dosiseinstellelement 16 mit. Insbesondere wird durch die Drehung des Dosiseinstellelements 16 der Dosisauswahlanschlag 100 relativ zu dem ersten und zweiten Dosieranschlag 110, 111 und den Mitnahmeanschlägen 11e verdreht. In der ersten Drehposition des Dosiseinstellelements 16 sind der Dosisauswahlanschlag 100 und der erste Dosieranschlag 110 und der dem ersten Dosieranschlag 110 zugeordnete, vorgelagerte Mitnahmeanschlag 11e in einer Flucht entlang der Längsachse L angeordnet. In der zweiten Drehposition des Dosiseinstellelements 16 ist der Dosisauswahlanschlag 100 um die Längsachse L winkelversetzt zu dem ersten Dosieranschlag 110 und in einer Flucht mit dem zweiten Dosieranschlag 111 und dem dem zweiten Dosieranschlag 111 zugeordneten, vorgelagerten Mitnahmeanschlag 11e angeordnet.

Zum Auslösen der Verabreichung der eingestellten Produktdosis, d. h. zum Lösen des Eingriffs des Eingriffselements 6a aus der Ausnehmung 7a des Stössels 7 wird die Nadelschutzhülse 3 um den Betätigungshub H_{B} in die proximale Richtung in das Gehäuse 2 verschoben. Hierdurch nimmt das proximale Ende 3a der Nadelschutzhülse 3 die Schalthülse 15 während eines ersten und zweiten Teilhubs, in den sich der Betätigungshub H_{B} untergliedert, mit.

Die Sperrhülse 8 weist einen Halteschnapper 8e auf, der in der Ausgangsposition der Nadelschutzhülse 3 axialfest in die Kupplungshülse 22, insbesondere in eine erste Rastausnehmung eingreift. Der Halteschnapper 8e ist quer zu Längsachse L aus der ersten Rastausnehmung bewegbar, wird jedoch von einer Haltefläche der Schalthülse 15 gehindert, sich aus der ersten Rastausnehmung zu bewegen (Figuren 14b, 16).

Während des ersten Teilhubs wird die Schalthülse 15 relativ zu der Sperrhülse 8 und der Kupplungshülse 22 bewegt, wodurch eine Ausnehmung 15a in Bezug auf die Längsachse L auf die Position des Halteschnappers 8e bewegt wird, so dass die laterale Blockierung des Halteschnappers 8e aufgehoben ist. Während des zweiten Teilhubs nimmt die Nadelschutzhülse 3 auch die Sperrhülse 8 mit. Die Sperrhülse 8 bewegt sich in die proximale Richtung relativ zu der Kupplungshülse 22. Ein Anschlag 3c der Nadelschutzhülse 3 stösst gegen ein Verriegelungsglied 8a der Sperrhülse 8 und nimmt die Sperrhülse 8 mit. Der Halteschnapper 8e rastet aus der ersten Rastausnehmung aus und in eine zweite Rastausnehmung der Kupplungshülse 22 ein. Hierdurch wird eine Verriegelung der Nadelschutzhülse 3 vorbereitet. Während des Betätigungshubs H_{B} wird eine zweite Feder 10, insbesondere Nadelschutzfeder, gespannt.

Durch die Bewegung der Sperrhülse 8 in die proximale Richtung wird die Haltefläche 8d entlang der Längsachse L aus der lateralen Position, in der sie die Bewegung des Eingriffselements 6a aus der Ausnehmung 7a blockiert, verschoben. Hierdurch kann die vorgespannte Ausschüttfeder 9 den Stössel 7 um den Ausschütthub H_{A} in die distale Richtung verschieben. Das Eingriffselement 6a wird aus dem Eingriff mit der Ausnehmung 7a mit einer Bewegung quer zur Längsachse L (Figur 19a) gedrückt.

Während des Ausschütthubs H_{A} wird der Stössel 7 soweit in die distale Richtung verschoben, dass der drehfeste Eingriff zwischen Stössel 7 und Kupplungsglied 22 gelöst wird.

Der Dosisauswahlanschlag 100 wird zu dem zweiten Dosieranschlag 111 hin bewegt, wobei der Dosisauswahlanschlag 100 gegen Ende des Ausschütthubs H_{A} den dem zweiten Dosieranschlag 111 zugeordneten und vorgelagerten Mitnahmeanschlag 11e zumindest ein Stück weit mitnimmt, wodurch das Verschiebeglied 11 relativ zu der Dosierhülse 5 in die distale Richtung verschoben wird bis der Dosisauswahlanschlag 100 an dem zweiten Dosieranschlag 111 anstösst, wodurch die Bewegung des Stössels 7 in die distale Richtung blockiert wird. Während des Ausschütthubs H_{A} wird der Dosisauswahlanschlag 100 an dem ersten Dosieranschlag 110 vorbeibewegt.

Durch die Bewegung des Verschiebeglieds 11 in die distale Richtung gleitet es am Ring 21 ab und dreht ihn aus seiner ersten Position (Figur 20), wodurch der Ring 21 von der Feder 20 in seine zweite Position gedrückt wird, und dabei einerseits ein Signal erzeugt und andererseits das Kupplungsglied 22 drehfest mit dem Dosierglied 5 kuppelt, wodurch das Dosiseinstellelement 16 relativ zu dem Gehäuse 2 verdrehfest ist und somit keine Dosiseinstellung mehr vorgenommen werden kann (Figur 21).

Beim Abnehmen des Autoinjektors von der Einstichstelle, verschiebt die zweite Feder 10 die Schalthülse 15 relativ zu dem Gehäuse 2 und/oder der Sperrhülse 8 in die distale Richtung, wobei die Schalthülse 15 die Nadelschutzhülse 3 in die distale Richtung um den Nadelschutzhub H_{N} verschiebt, wodurch das Verriegelungsglied 8a in die Schalthülse 15, insbesondere die Ausnehmung 15a einrastet, und die Schalthülse 15 das Ausrasten des Halteschnappers 8e aus der zweiten Rastausnehmung der Kupplungshülse 22 blockiert.

Durch den Eingriff des Verriegelungsglieds 8a in die Ausnehmung 15a und den Eingriff des Halteschnappers 8e in die Kupplungshülse 22 werden die Sperrhülse 8 und die Schalthülse 15 an einer Bewegung in die distale Richtung gehindert, so dass beim Versuch die Nadelschutzhülse 3 in die proximale Richtung zu verschieben, diese Bewegung blockiert wird.

### Dritte Ausführungsform

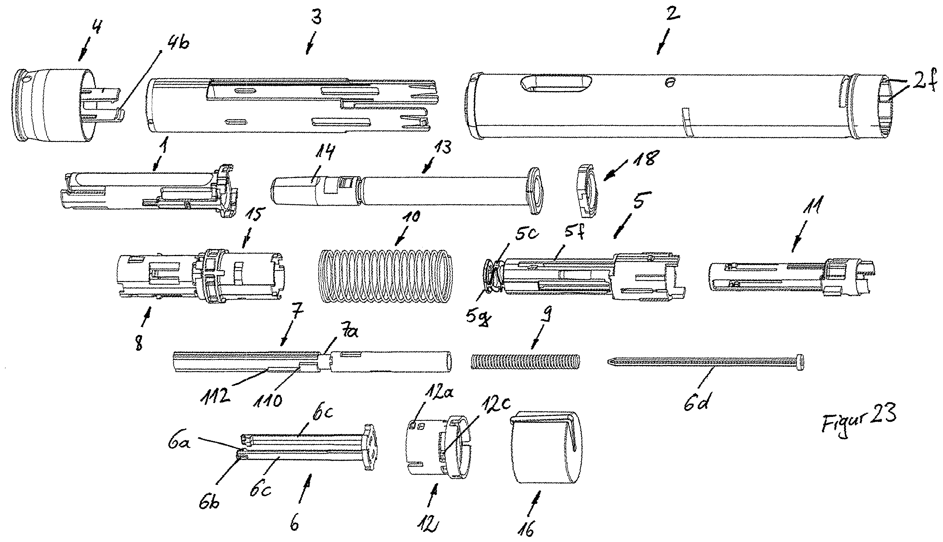

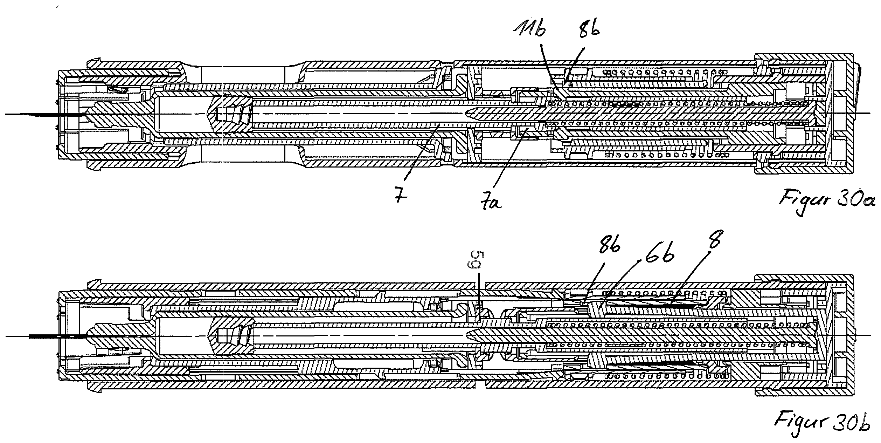

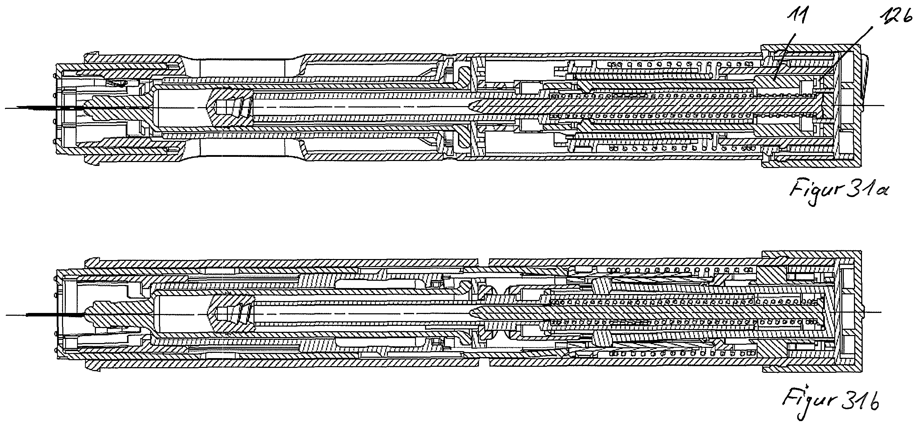

Bezugnehmend auf die Figuren 23 bis 32b werden nun die strukturellen Merkmale und die Funktion des Autoinjektors gemäss der dritten Ausführungsform beschrieben.

Der Autoinjektor weist ein hülsenförmiges, längliches Gehäuse 2 mit einer Längsachse L auf, welches an seinem proximalen Ende eine Rotationshülse 12 aufweist, die formschlüssig mit dem Gehäuse 2 drehbar und axialfest verbunden ist und am proximalen Ende des Gehäuses 2 angeordnet ist. Die Rotationshülse 12 ist mittels eines Vorsprungs 12a mit einer Ausnehmung 2a des Gehäuses 2 so verschnappt, dass sie relativ zu dem Gehäuse 2 drehbar und axialfest und insbesondere nicht oder nicht ohne weiteres von dem Gehäuse 2 lösbar ist. Die Rotationshülse 12 weist einen Rastschnapper 12c auf. Das Gehäuse 2 weist mehrere, insbesondere mindestens zwei, über den Umfang verteilte Rastnuten 2f auf, wobei der Rastschnapper 12c in eine der Rastnuten 2f eingreift und bei einer Drehung der Rotationshülse 12, wie z.B. für eine Dosiseinstellung, aus der Rastnut 2f bewegt und in eine andere der Rastnuten 2f bewegt wird. Hierdurch werden quasistabile Rastpositionen für die Rotationshülse 12 und ein mit der Rotationshülse 12 drehfest verbundenes Dosiseinstellelement 16 vorgegeben.

Das hülsenförmige Dosiseinstellelement 16 ist am proximalen Ende des Gehäuses 2 drehbar und axialfest mit dem Gehäuse 2 verbunden und bildet insbesondere das proximale Ende des Autoinjektors. Das Dosiseinstellelement 16 ist vom Verwender der Vorrichtung greifbar und relativ zu dem Gehäuse 2 für die Einstellung einer auszuschüttenden Produktdosis drehbar. Beispielsweise sind zwei unterschiedliche auszuschüttende Dosen einstellbar, wie z.B. 0,25 und 0,5 ml. Alternativ kann der Rastschnapper 12c an dem Dosiseinstellelement 16 angeordnet sein. Beispielsweise kann das Dosiseinstellelement 16 in Bezug auf das Gehäuse 2 mindestens drei unterschiedliche quasistabile Drehpositionen einnehmen, wobei in einer Ausgangsposition keine Dosis, in der ersten Position eine erste Dosis und in der zweiten Position eine zweite Dosis eingestellt sein kann, usw.

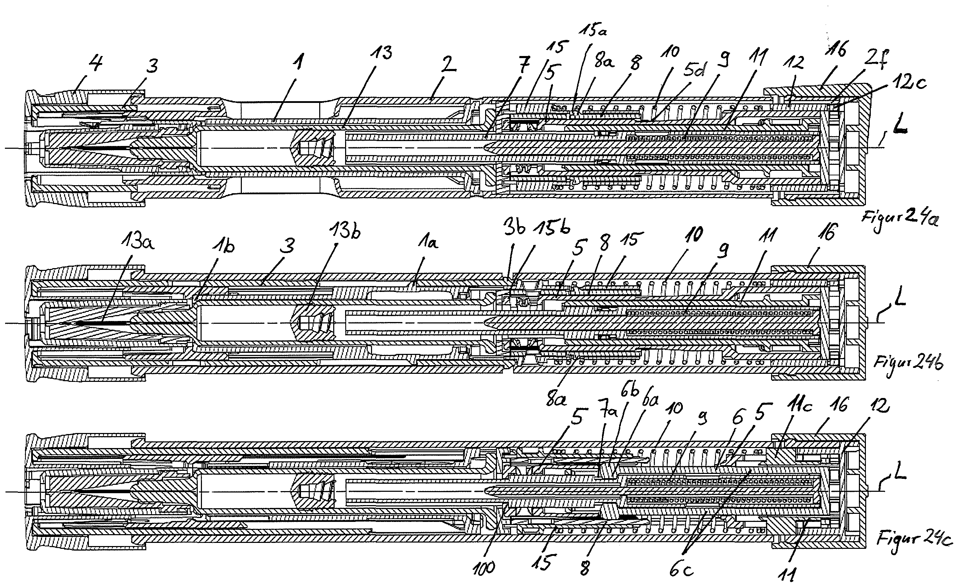

An dem distalen Ende des Autoinjektors ist in seinem Auslieferungszustand (Figuren 24a-24c) eine Abziehkappe 4 angeordnet, die vor der Verwendung des Autoinjektors abgezogen, oder abgedreht, und entfernt wird.

In dem Gehäuse 2 ist ein Produktbehälter 13 in der Gestalt einer Spritze in Bezug auf das Gehäuse 2 - abgesehen von der Montage des Autoinjektors - entlang der Längsachse L unverschiebbar aufgenommen. Der Produktbehälter 13 weist einen hülsenförmigen Spritzenkörper auf, der einen Kolben 13b, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel 13a auf, deren distales Ende von der Nadelspitze gebildet wird. Zwischen der Injektionsnadel 13a und dem Kolben 13b ist ein flüssiges Produkt, insbesondere Medikament, innerhalb des Spritzenkörpers angeordnet, wobei das flüssige Produkt durch Verschieben des Kolbens 13b in eine Ausschüttrichtung, d. h. in distale Richtung oder zu der Injektionsnadel 13a hin, das Produkt durch die hohle Injektionsnadel 13a aus dem Produktbehälter 13 ausschüttet. Der Spritzenkörper weist an seinem proximalen Ende einen sogenannten Fingerflansch auf, der radial nach aussen über den Aussenumfang des zylindrischen Spritzenkörpers ragt.

Der Produktbehälter 13 ist in einem Produktbehälterhalter 1, der im Folgenden als Spritzenhalter 1 bezeichnet wird, so aufgenommen, dass er zumindest gegen eine Bewegung entlang der Längsachse L in die distale Richtung relativ zu dem Spritzenhalter 1 gesichert ist. Der Spritzenhalter 1 ist, wie z.B. aus Figur 24a ersichtlich ist, mit dem Gehäuse 2 formschlüssig verbunden, insbesondere verrastet. Das Gehäuse 2 weist hierzu Ausnehmungen auf, in die Rastelemente, die hier am proximalen Ende des Spritzenhalters 1 gebildet werden, eingreifen. Der Spritzenhalter 1 weist mindestens eine nach innen ragende Schulter 1b auf, an der sich ein verjüngender Abschnitt des Produktbehälters 13, der distal des zylindrischen Spritzenkörperabschnitts, welcher den Kolben 13b führt, angeordnet ist, abstützt.

Der Autoinjektor weist eine in dem Gehäuse 2 angeordnete Dosierhülse 5 auf, die relativ zu dem Gehäuse 2 und einem Stössel 7 drehbar und bevorzugt axialfest, insbesondere axialfest mit dem Gehäuse 2 oder einem hülsenförmigen Gehäuseeinsatz verschnappt, ist.

Um zu verhindern, dass der Produktbehälter 13 relativ zu dem Spritzenhalter 1 in die proximale Richtung verschiebbar ist, wird der Produktbehälter 13 an seinem proximalen Ende durch einen über eine Verdrehsicherungshülse 18 auf den Spritzenkörper wirkenden Haltefederabschnitt 5c in den Eingriff mit der Schulter 1b gedrückt. Durch den Haltefederabschnitt 5c können Längenunterschiede des Produktbehälters 13, die aufgrund von Herstellungstoleranzen entstehen können, ausgeglichen werden, wobei der feste Sitz des Produktbehälters 13 an der Schulter 1b sichergestellt ist.

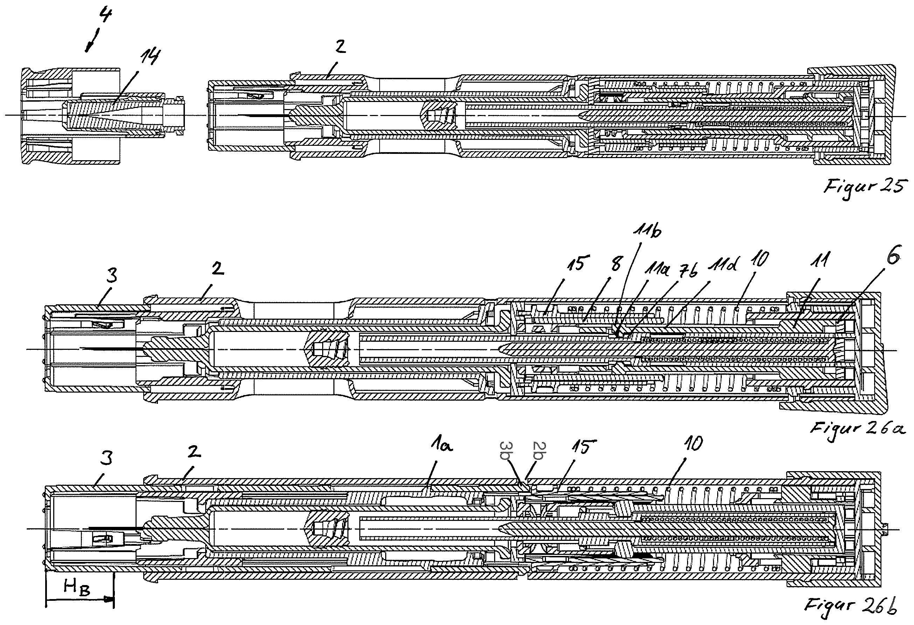

Der Produktbehälter 13 ist in Bezug auf das Gehäuse 2 so angeordnet, dass die Nadelspitze distal über das distale Ende des Gehäuses 2 steht. Im Ausgangs- oder Auslieferungszustand des Autoinjektors, d. h., wenn die Abziehkappe 4 an dem Autoinjektor angeordnet ist, wird die Nadel 13a von einer Nadelabdeckkappe 14, die in dem gezeigten Beispiel als sogenanntes und dem Fachmann bekanntes rigid needle shield, alternativ als soft needle shield, ausgebildet ist, abgedeckt, um die Nadel 13a vor Verschmutzung zu schützen bzw. die Nadel 13a und das Medikament steril zu halten. Das rigid needle shield 14 ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter 1b ist zwischen dem Spritzenkörper und dem proximalen Ende des rigid needle shield 14 angeordnet, insbesondere so, dass zwischen dem rigid needle shield 14 und der Schulter 1b ein - wenngleich auch geringer - Spalt entsteht, um zu verhindern, dass die Schulter 1b eine Kraft auf das rigid needle shield 14 ausübt, wodurch z. B. die Sterilität der Nadel 13a oder des flüssigen Produkts gefährdet werden könnte. Die Abziehkappe 4 ist mit dem Gehäuse 2 oder einer Nadelschutzhülse 3 lösbar verschnappt, wobei diese Verschnappung gelöst wird, wenn die Abziehkappe 4 von dem Gehäuse 2 oder der Nadelschutzhülse 3 entfernt wird. Die Abziehkappe 4 weist ferner insbesondere an einem flexiblen Arm mindestens einen Schnapper 4b auf, der in eine Lücke zwischen dem Spritzenkörper, insbesondere dessen sich verjüngenden Bereichs, und dem proximalen Ende des rigid needle shield 14 eingreift. Wenn die Abziehkappe 4 von dem Autoinjektor entfernt wird, hakt der Schnapper 4b in das proximale Ende des rigid needle shield 14 ein, wodurch das rigid needle shield 14 von dem Produktbehälter 13 gelöst und zusammen mit der Abdeckkappe 4 von dem Autoinjektor entfernt wird (Figur 25).

Der Autoinjektor weist eine Nadelschutzhülse 3 auf, die relativ zu dem Gehäuse 2 und entlang der Längsachse L um einen Betätigungshub H_{B} in die proximale Richtung in eine betätigte Position verschiebbar ist, um eine Produktausschüttung auszulösen. In der Ausgangsposition der Nadelschutzhülse 3, wie sie in den Figuren 26a-26b gezeigt wird, wobei die Abziehkappe 4 abgenommen ist, steht das distale Ende der Nadelschutzhülse 3 distal über die Nadelspitze der Nadel 13a, so dass ein Zugriff auf die Nadelspitze zunächst verhindert wird. Ein Schnapper 3b an der Nadelschutzhülse 3 ist in einer Ausnehmung 2b des Gehäuses eingerastet. Durch Verschieben der Nadelschutzhülse 3 (Figuren 26b-27a) um den Betätigungshub H_{B} wird dabei der Schnapper 3b aus der Ausnehmung 2b des Gehäuses 2 heraus gedrückt und die Nadelschutzhülse 3 wird soweit in die proximale Richtung verschoben, dass die Nadel 13a aus dem distalen Ende der Nadelschutzhülse 3 hervortritt, insbesondere mit einer Länge hervortritt, welche der Injektionstiefe der Nadel in die Einstichstelle entspricht. Bevorzugt soll die Nadel 13a soweit über das distale Ende der Nadelschutzhülse 3 stehen, dass eine subkutane Injektion erfolgen kann. Insbesondere kann das Gehäuse 2 einen Anschlag bilden, an dem die Nadelschutzhülse 3 in der betätigten Position anliegt.

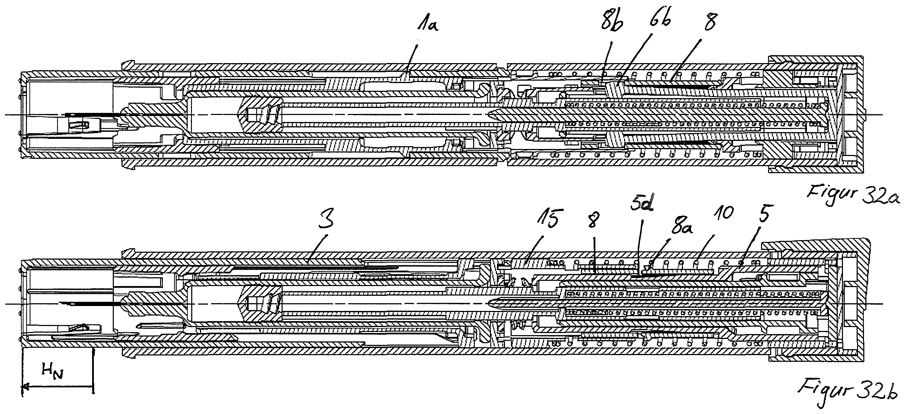

Nach der erfolgten Injektion kann die Nadelschutzhülse 3 relativ zu dem Gehäuse 2 aus der betätigten Position entlang der Längsachse L um einen Nadelschutzhub H_{N} in die distale Richtung in eine Nadelschutzposition (Figuren 32a-32b) verschoben werden. In der Nadelschutzposition steht das distale Ende der Nadelschutzhülse 3 distal über die Nadelspitze, so dass ein Zugriff auf die Nadelspitze verhindert und eine Verletzungsgefahr verringert wird. Die Nadelschutzhülse 3 kann, wie weiter unten beschrieben wird, gegen erneutes Zurückschieben aus der Nadelschutzposition blockiert werden.

Der Spitzenhalter 1 weist eine Abragung 1a, die radial nach aussen weist, auf, wobei die Abragung 1a in eine schlitzförmige Ausnehmung der Nadelschutzhülse 3, die zwischen dem Gehäuse 2 und dem Spritzenhalter 1 angeordnet ist, eingreift. In der Ausgangsposition der Nadelschutzhülse 3 (Figuren 24a-24c) und/oder in der Nadelschutzposition der Nadelschutzhülse 3 (Figuren 32a-32b) liegt die Nadelschutzhülse 3, insbesondere das proximale Ende der schlitzförmigen Ausnehmung an der Abragung 1a an, wodurch eine Bewegung der Nadelschutzhülse 3 in die distale Richtung verhindert wird. In diese schlitzförmige Ausnehmung, alternativ in eine andere Ausnehmung der Nadelschutzhülse 3, kann eine Nocke, die federnd an dem Spritzenhalter 1 angeordnet und von dem Spritzenhalter 1 gebildet ist, eingreifen. Der Schnapper 3b an der Nadelschutzhülse 3 ist in der Ausnehmung 2b des Gehäuses so eingerastet, dass bei dem Versuch, die Nadelschutzhülse 3 aus der Ausgangsposition in die betätigte Position zu verschieben, der Schnapper 3b die Verschiebung der Nadelschutzhülse 3 zunächst verhindert, wobei der Schnapper 3b aus der Ausnehmung 2b herausgedrückt wird, wenn die auf die Nadelschutzhülse 3 zum Zurückschieben aufgebrachte Kraft einen gewissen Schwellwert überschreitet, wodurch die Nadelschutzhülse 3 schlagartig in die betätigte Position zurückgeschoben wird (Figuren 26b-27a). Die Nadel 13a kann hierdurch schlagartig in die Einstichstelle eingestochen werden. Zum Einstechen der Nadel 13a bzw. zum Verschieben der Nadelschutzhülse 3 in die betätigte Position wird das distale Ende der Nadelschutzhülse 3 an die Einstichstelle gesetzt, wobei das Gehäuse 2 dann in Richtung Einstichstelle gedrückt wird, wobei wenn die Andrückkraft den oben genannten Schwellwert überschreitet, das Gehäuse 2 schlagartig zu der Einstichstelle hin und die Nadelschutzhülse 3 relativ zu dem Gehäuse 2 um den Betätigungshub H_{B} in die betätigte Position verschoben wird.

Der Autoinjektor weist ferner einen hülsenförmigen Stössel 7 auf, welcher eine nach innen ragende Schulter bildet, an der sich eine erste Feder 9, die auch als Ausschüttfeder bezeichnet werden kann, abstützt. Die erste Feder 9 ist innerhalb des hülsenförmigen Stössels 7 angeordnet. Die erste Feder 9 ist eine als Druckfeder wirkende Wendelfeder, die im Ausgangs- oder Auslieferungszustand des Autoinjektors mit so viel Energie vorgespannt ist, dass sie das in dem Produktbehälter 13 enthaltene Produkt insbesondere vollständig durch Verschieben des Stössels 7 um einen Ausschütthub H_{A} aus dem Produktbehälter 13 ausschütten kann. Im Auslieferungszustand der Vorrichtung besteht zwischen dem Kolben 13b und dem distalen Ende des Stössels 7 ein Abstand, so dass der Stössel 7 erst während der Ausführung des Ausschütthubs H_{A} an den Kolben 13b anschlägt und diesen in die Ausschüttrichtung mitnimmt.

Die erste Feder 9 stützt sich mit ihrem proximalen Ende an einem Halteelement 6 ab, welches in diesem Beispiel zwei Arme 6c aufweist, wobei an jedem Arm 6c ein erstes Eingriffselement 6a und ein zweites Eingriffselement 6b angeordnet ist. Das erste Eingriffselement 6a weist radial zu der Längsachse L hin, wobei das zweite Eingriffselement 6b radial von der Längsachse L weg weist. Das erste Eingriffselement 6a greift in eine erste Ausnehmung 7a, die von dem Stössel 7 gebildet wird, ein, wodurch eine Bewegung des Stössels 7 relativ zu dem Halteelement 6 in die distale Richtung oder in die Ausschüttrichtung verhindert wird. Hierdurch wird die erste Feder 9 in ihrem gespannten Zustand gehalten. Innerhalb der ersten Feder 9 ist ein Führungsstift 6d angeordnet, der durch das proximale Ende der ersten Feder 9 in die Seele der Feder 9 eingefügt ist und der an seinem proximalen Ende einen Kopf aufweist, an dem sich die erste Feder 9 mit ihrem proximalen Ende abstützt. Der Kopf des Führungsstifts 6d liegt lose an dem Halteelement 6 an, so dass das Halteelement 6 relativ zu dem Führungsstift 6d drehbar ist. Der Führungsstift 6d verhindert ein seitliches Ausknicken der ersten Feder 9 während und am Ende des Ausschütthubs H_{A} des Stössels 7.

Der Autoinjektor weist ein Schaltmodul 8, 15 auf, welches eine Schalthülse 15 und eine von der Schalthülse 15 umgebene Sperrhülse 8 aufweist. Im Auslieferungszustand der Vorrichtung wird das erste Eingriffselement 6a von dem Innenumfang der Sperrhülse 8, der an dem zweiten Eingriffselement 6b anliegt, in dem Eingriff mit der ersten Ausnehmung 7a gehalten.

Die Schalthülse 15 ist mit dem proximalen Ende 3a der Nadelschutzhülse 3 verbunden oder liegt zumindest an dem proximalen Ende 3a der Nadelschutzhülse 3 an. Eine zweite Feder 10, innerhalb der die erste Feder 9 angeordnet ist und die vorzugsweise die Schalthülse 15 und die Sperrhülse 8 zumindest teilweise umgibt, stützt sich mit ihrem distalen Ende an der Schalthülse 15 ab. Ein Teil der Schalthülse 15 ist somit zwischen der Nadelschutzhülse 3 und dem distalen Ende der zweiten Feder 10 angeordnet. Die zweite Feder 10 ist eine als Druckfeder wirkende und als Wendelfeder ausgestaltete Feder aus Metall. Die zweite Feder 10 stützt sich mit ihrem proximalen Ende an einem Verschiebeglied 11, insbesondere an einer Abragung 11c, die axial verschiebbar und verdrehfest in das Dosierhülse 5 eingreift, insbesondere durch eine schlitzförmige Nut 5b der Dosierhülse 5 hindurchgreift, ab. Die zweite Feder 10 umgibt somit auch die Dosierhülse 5 zumindest teilweise, vorzugsweise vollständig.

Das Schaltglied 15 weist eine Ausnehmung 15a auf, in welche ein Verriegelungsglied 8a der Sperrhülse 8 eingreift. Das Verriegelungsglied 8a ist sägezahnförmig und ragt radial von der Längsachse L weg. Das Verriegelungsglied 8a ist federnd an einem Arm, welcher von der Sperrhülse 8 gebildet wird, angeordnet. Durch Verschieben der Schalthülse 15 in die proximale Richtung wird die Sperrhülse 8 über den Eingriff des Verriegelungsglieds 8a in die proximale Richtung mitgenommen.

Durch Verschieben der Nadelschutzhülse 3 in die betätigte Position wird die Schalthülse 15 ebenfalls um den Betätigungshub H_{B} mitgenommen, wodurch die zweite Feder 10 gespannt wird. Wird die Nadelschutzhülse 3 nicht vollständig in die betätigte Position verschoben, kann die zweite Feder 10 die Schalthülse 15 und die Nadelschutzhülse 3 wieder zurück in die Ausgangsposition verschieben, wobei über den Eingriff des Verriegelungsglieds 8a auch die Sperrhülse 8 von der Schalthülse 15 mitgenommen wird.

Das insbesondere hülsenförmige Verschiebeglied 11 ist im Auslieferungszustand oder vor der Auslösung der Produktausschüttung in einem axialfesten Eingriff mit dem Stössel 7. Das Verschiebeglied 11 weist ein erstes Eingriffsglied 11a, welches in eine Ausnehmung 7b des Stössels 7 eingreift, und ein zweites Eingriffsglied 11b auf. Das erste Eingriffsglied 11a und das zweite Eingriffsglied 11b sind an dem Ende eines Arms 11d federnd angeordnet. Das Verschiebeglied 11 weist zwei solcher Arme 11d mit einem ersten Eingriffsglied 11a und einem zweiten Eingriffsglied 11b auf. Das erste Eingriffsglied 11a weist radial zu der Längsachse L hin, wobei das zweite Eingriffsglied 11b radial von der Längsachse L weg weist. Im Auslieferungszustand wird das erste Eingriffsglied 11a von dem Innenumfang der Sperrhülse 8 in dem axialfesten Eingriff mit dem Stössel 7 gehalten. Das zweite Eingriffsglied 11b liegt an dem Innenumfang der Schalthülse 8 an. Die Rotationshülse 12 weist einen Signalanschlag 12b auf, an den das Verschiebeglied 11 zur Erzeugung eines Signals anschlagen kann und vorzugsweise an dem das Verschiebeglied 11 im Auslieferungszustand der Vorrichtung anliegt.

Die entlang der Längsachse L vorzugsweise verschiebbare Verdrehsicherungshülse 18 verbindet das Gehäuse 2 und den Stössel 7 drehfest, wobei der Stössel 7 relativ zu der Verdrehsicherungshülse 18 entlang der Längsachse L verschiebbar ist. Der Stössel 7 weist an seinem Aussenumfang einen ersten Dosieranschlag 110 und optional einen Halteanschlag 112 auf.

Zumindest das Dosiseinstellelement 16 und die Dosierhülse 5 sind drehfest verbunden, so dass eine Drehung des Dosiseinstellelements 16 eine Drehung der Dosierhülse 5 bewirkt. In dem gezeigten Beispiel ist das Dosiseinstellelement 16 drehfest mit der Dosierhülse 5, dem Verschiebeglied 11, dem Haltelement 6, der Schalthülse 15, der Sperrhülse 8 und der zweiten Feder 10 verbunden, so dass eine Drehung des Dosiseinstellelements 16 eine Drehung der Dosierhülse 5, des Verschiebeglieds 11, des Haltelements 6, der Schalthülse 15, der Sperrhülse 8 und der zweiten Feder 10 bewirkt.

Die Dosierhülse 5, insbesondere ein das distale Ende der Dosierhülse 5 bildende Halteabschnitt 5g, der über den Haltefederabschnitt 5c federnd mit einem Hauptabschnitt 5f der Dosierhülse 5 monolithisch verbunden ist, weist einen Dosisauswahlanschlag 100 auf. In der Ausgangsposition, insbesondere wenn keine Dosis mittels des Dosiseinstellelements 16 eingestellt ist, kann der optional vorhandene Halteanschlag 112 an dem Dosisauswahlanschlag 100 anliegen, um die Ausschüttmechanik des Autoinjektors während längerer Lagerzeiten, von der Kraft der vorgespannten ersten Feder 9 zu entlasten.

Durch Drehen des Dosiseinstellelements 16 für die Einstellung der zu verabreichenden Produktdosis kann der Dosisauswahlanschlag 100 relativ zu dem ersten Dosieranschlag und dem optional vorhandenen Halteanschlag 112 um die Längsachse L verdreht werden.

In der Ausgangsposition des Autoinjektors greift ein Schnapper 3b der Nadelschutzhülse 3 in eine Ausnehmung des Gehäuses 2 ein (Figur 24b). Die Schalthülse 15 weist eine Haltefläche 15b auf, welche in Bezug auf den Schnapper 3b so angeordnet ist, dass sie den Schnapper 3b daran hindert sich aus der Ausnehmung zu bewegen. Um die Nadelschutzhülse 3 in die proximale Richtung verschieben zu können, ist es erforderlich, dass die Blockierung der Bewegung aus der Ausnehmung aufgehoben ist und sich der Schnapper 3b aus der Ausnehmung bewegen kann.

Zur Verabreichung des Produkts aus dem Produktbehälter 13 wird die Abziehkappe 4 von dem Autoinjektor zusammen mit dem rigid needle shield 14 entfernt (Figur 25). Anschliessend oder davor wird mittels Drehung des Dosiseinstellelements 16 relativ zu dem Gehäuse eine auszuschüttende Dosis eingestellt. Durch das Drehen des Dosiseinstellelements 16 aus der Ausgangsposition, in der der Halteanschlag 112 an dem Dosisauswahlanschlag 100 anliegt, in eine erste Drehposition für eine erste Dosis oder eine zweite Drehposition für eine zweite Dosis wird die Haltefläche 15b in Bezug auf den Schnapper 3b in eine Position verdreht, in welcher der Schnapper 3b sich aus der Ausnehmung bewegen kann.

Wenn das Dosiseinstellelement 16 seine erste Drehposition der mindestens zwei Drehpositionen einnimmt, ist der Dosisauswahlanschlag 100 in einer Flucht entlang der Längsachse L mit dem ersten Dosieranschlag 110 angeordnet.

Wenn das Dosiseinstellelement 16 seine zweite Drehposition der mindestens zwei Drehpositionen einnimmt, ist der Dosisauswahlanschlag 100 um die Längsachse L winkelversetzt zu dem ersten Dosieranschlag 110 angeordnet.

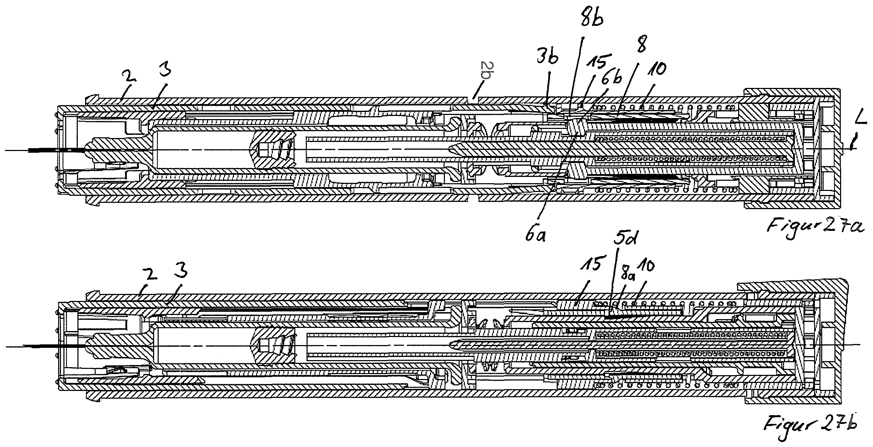

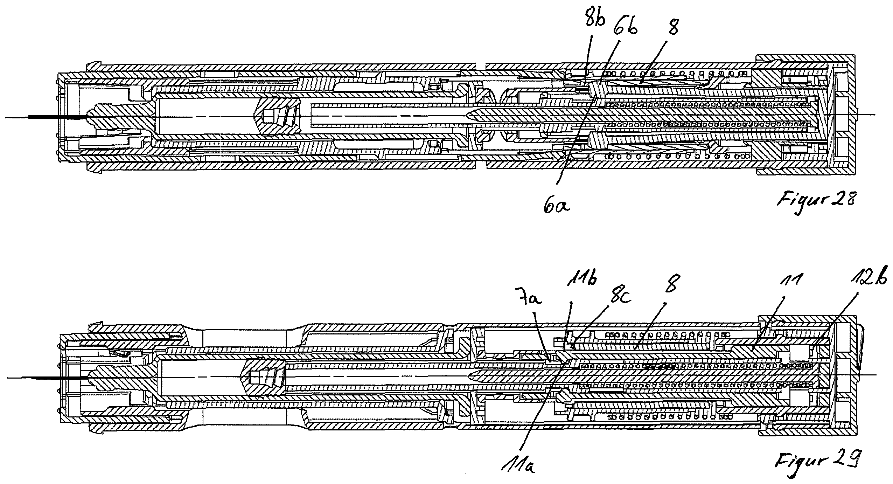

Das distale Ende der Nadelschutzhülse 3 wird an die Einstichstelle eines Patienten angesetzt, wobei das Gehäuse 2 zu der Einstichstelle hin verschoben wird, wodurch sich die Nadelschutzhülse 3 aus ihrer Ausgangsposition um den Betätigungshub H_{B} in die proximale Richtung relativ zu dem Gehäuse 2 in die betätigte Position bewegt (Figuren 26a,b und 27a,b). Dabei wird der Schnapper 3b aus der Ausnehmung des Gehäuses 2 gedrückt. Die zweite Feder 10 wird gespannt, wobei die Schalthülse 15 von der Nadelschutzhülse 3 um den Betätigungshub H_{B} mitgenommen wird. Die Sperrhülse 8 weist eine erste Ausnehmung 8b auf, die durch Verschieben der Sperrhülse 8 um den Betätigungshub H_{B} entlang der Längsachse L auf die Position des zweiten Eingriffselements 6b gebracht wird, wie in den Figuren 27a,b dargestellt. Hierdurch wird das erste Eingriffselement 6a aus dem Eingriff mit dem Stössel 7 mit einer Bewegung quer zu und von der Längsachse L weg bewegt, wobei gleichzeitig das zweite Eingriffselement 6b in den Eingriff mit der Sperrhülse 8, insbesondere deren erster Ausnehmung 8b bewegt wird. Hierdurch wird der Stössel 7 für die Bewegung um den Ausschütthub H_{A} in die Ausschüttrichtung freigegeben (Figur 28), wobei die Grösse des Ausschütthubs H_{A} von der Drehposition des Dosiseinstellelements 16 abhängt.

Wenn das Dosiseinstellelement 16 in seiner ersten Drehposition ist, wird der erste Dosieranschlag 110 zu dem Dosisauswahlanschlag 100 hin bewegt bis er an dem Dosisauswahlanschlag 100 anstösst, wodurch die Ausschüttbewegung des Stössels 7 gestoppt und nur eine echte Teilmenge des Produkts im Produktbehälter 13 ausgestossen wird.

Wenn das Dosiseinstellelement 16 in seiner zweiten Drehposition ist, wird der erste Dosieranschlag 110, der um die Längsachse L winkelversetzt zu dem Dosisauswahlanschlag 100 angeordnet ist, an dem Dosisauswahlanschlag 100 entlang der Längsachse L vorbeibewegt. Hierbei ist der Ausschütthub H_{A} grösser als in der ersten Drehposition des Dosiseinstellelements 16. In dem gezeigten Beispiel wird der Kolben 13b soweit im Produktbehälter 13 verschoben, bis er an dem sich verjüngenden Bereich des Spritzenkörpers anstösst, wodurch die Ausschüttbewegung des Stössels 7 gestoppt wird bzw. die gesamte im Produktbehälter 13 enthaltene Produktmenge ausgestossen wird. Alternativ kann am Stössel ein zweiter Dosieranschlag (nicht gezeigt) gebildet sein, der in Bezug auf den ersten Dosieranschlag 110 um Längsachse L drehwinkelversetzt und entlang der Längsachse L auf einer anderen Position angeordnet ist, wobei in der zweiten Drehposition des Dosiseinstellelements 16 der Dosisauswahlanschlag 100 in einer Flucht entlang der Längsachse L mit dem zweiten Dosieranschlag angeordnet ist. Der zweite Dosieranschlag wird dann zu dem Dosisauswahlanschlag 100 hin bewegt bis er an dem Dosisauswahlanschlag 100 anstösst, wodurch die Ausschüttbewegung des Stössels 7 gestoppt und nur eine echte Teilmenge, die grösser als die Teilmenge, wenn sich das Dosiseinstellelement in seiner ersten Drehposition befindet, ist, ausgestossen wird. Analog zu dieser Alternative kann in einer Weiterbildung das Dosiseinstellelement 16 in eine dritte Drehposition gedreht werden, in der ein dritter Dosieranschlag und der Dosisauswahlanschlag 100 fluchten, usw.

Wenn der Stössel 7 für die Bewegung um den Ausschütthub H_{A} in die Ausschüttrichtung freigegeben ist (Figur 28), ist die axialfeste Kopplung zwischen dem Stössel 7 und dem Halteelement 6 aufgehoben, wodurch das Halteelement 6, welches zumindest ein Stück weit relativ zu dem Gehäuse 2 und entlang der Längsachse L bewegbar ist, von der ersten Feder 9 in die proximale Richtung bewegt werden kann, wobei das Halteelement 6 über den Eingriff des zweiten Eingriffselements 6b in die Ausnehmung 8b die Sperrhülse 8 um einen Startsignalhub mitnimmt, wodurch die Sperrhülse 8 an einem Startsignalanschlag, der von der Dosierhülse 5 gebildet wird, anschlägt und hierdurch ein akustisches und/oder taktiles Signal ausgibt, welches dem Anwender der Vorrichtung signalisiert, dass die Produktausschüttung gestartet wurde. Durch die Verschiebung der Sperrhülse 8 um den Betätigungshub H_{B} wird das Verriegelungsglied 8a für eine Bewegung quer und zu der Längsachse L hin freigegeben, da die Dosierhülse 5 eine Vertiefung 5d aufweist, welche eine solche Bewegung des Verriegelungsglieds 8a zulässt, wenn die Sperrhülse 8 um den Betätigungshub H_{B} verschoben wurde oder wenn die Nadelschutzhülse 3 in ihrer betätigten Position ist (Figur 27b).

Da das Verschiebeglied 11 noch axialfest mit dem Stössel 7 verbunden ist, wird es um einen ersten Teilhub des Ausschütthubs H_{A} in die Ausschüttrichtung mitgenommen, wobei das Verschiebeglied 11 in etwa um den ersten Teilhub von dem Signalanschlag 12b weg bewegt wird (12b), wie am besten aus Figur 29 erkennbar ist. Am Ende des ersten Teilhubs, während dem das erste und zweite Eingriffsglied 11a, 11b relativ zu der Sperrhülse 8 bewegt werden, wird das erste Eingriffsglied 11a aus dem Eingriff mit dem Stössel 7 gedrückt, wobei gleichzeitig das zweite Eingriffsglied 11b in die zweite Ausnehmung 8c der Sperrhülse 8 mit einer Bewegung quer zu der Längsachse L und radial von der Längsachse L weg bewegt wird (Figur 29). Hierdurch wird das Verschiebeglied 11 daran gehindert, sich in die proximale Richtung relativ zu dem Gehäuse 2 oder der Sperrhülse 8 zu bewegen. Das zweite Eingriffsglied 11b wird von dem Aussenumfang des Stössels 7 in den Eingriff mit der Ausnehmung 8c gehalten, wenn der Stössel 7 um seinen zweiten Teilhub des Ausschütthubs H_{A} bewegt wird. Die Aussenumfangsfläche des Stössels 7 hält das zweite Eingriffselement 6b in dem Eingriff mit der ersten Ausnehmung 8b der Sperrhülse 8. Am Ende des Ausschütthubs H_{A} gibt das Vortriebsglied 7 das zweite Eingriffsglied 11b aus dem Eingriff mit der Sperrhülse 8 frei, wodurch das zweite Eingriffsglied 11b aus dem Eingriff mit der Ausnehmung 8c bewegt wird, insbesondere zu der Längsachse L hin, so dass die zweite Feder 10 das Verschiebeglied 11 entgegen die Ausschüttrichtung, d. h. in die proximale Richtung beschleunigt, so dass beim Auftreffen des Verschiebeglieds 11 auf dem Signalanschlag 12b ein akustisches und/oder taktiles Signal erzeugt wird.

Wie am besten aus Figur 32a erkennbar ist, bleibt der Eingriff des zweiten Eingriffselements 6b in die erste Ausnehmung 8b bestehen, wodurch eine Bewegung der Sperrhülse 8 in die distale Richtung relativ zu dem Gehäuse 2 verhindert wird.

Durch Abnehmen des Autoinjektors von der Injektionsstelle kann die zweite Feder 10 die Schalthülse 15 und die Nadelschutzhülse 3 aus der betätigten Position in die Nadelschutzposition um den Nadelschutzhub H_{N} bewegen, wobei das Verriegelungsglied 8a aus dem Eingriff mit der Ausnehmung 15a gedrückt wird, wobei sich die Schalthülse 15 relativ zu der Sperrhülse 8 in die distale Richtung bewegt. Wenn die Nadelschutzhülse 3 in ihrer Nadelschutzposition ist, verschnappt das Verriegelungsglied 8a mit der Schalthülse 15, wobei das Verriegelungsglied 8a ein Zurückschieben der Nadelschutzhülse 3 in ihre betätigte Position verhindert. Bei dem Versuch, die Nadelschutzhülse 3 aus der Nadelschutzposition in die betätigte Position zurückzuschieben, stösst das Schaltglied 15 an dem Verriegelungsglied 8a an, welches die Bewegung der Nadelschutzhülse 3 in die betätigte Position verhindert. Die Sperrhülse 8 stützt sich hierzu axial an dem Startsignalanschlag der Dosierhülse 5 ab.

**Bezugszeichenliste**

| | | | |
|---|---|---|---|
| 1 | Spritzenhalter | 10 | zweite Feder/Nadelschutzfeder |
| la | Abragung/ Halteglied | | |
| 1b | Schulter/ Eingriffsglied | 11 | Verschiebeglied |
| 1c | Nocke | 11a | erstes Eingriffsglied |
| | | 11b | zweites Eingriffsglied |
| 2 | Gehäuse | 11c | Abragung |
| 2a, 2b | Ausnehmung | 11d | Arm |
| 2f | Rastnut | 11e | Mitnahmeanschlag |
| | | | |
| | | 12 | Rotationshülse / hülsenförmiger Gehäuseeinsatz |
| 3 | Nadelschutzhülse | | |
| 3a | proximales Ende | 12a | Vorsprung |
| 3b | Schnapper | 12b | Signalanschlag |
| 3c | Anschlag | 12c | Rastschnapper |
| | | 12d | Haltefläche |
| 4 | Abziehkappe | | |
| 4b | Schnapper | 13 | Produktbehälter/Spritze |
| | | 13a | Nadel |
| 5 | Dosierhülse | 13b | Kolben |
| 5c | Haltefederabschnitt | | |
| 5d | Vertiefung | 14 | rigid needle shield/ Nadelschutzkappe |
| 5e | Stoppanschlag | | |
| 5f | Hauptabschnitt | | |
| 5g | Halteabschnitt | 15 | Schalthülse |
| | | 15a | Ausnehmung |
| 6 | Halteelement | 15b | Haltefläche |
| 6a | erstes Eingriffselement | | |
| 6b | zweites Eingriffselement | 16 | Dosiseinstellelement |
| 6c | Arm | | |
| 6d | Führungsstift | 17 | Mechanikhalter |
| | | 17a | Stoppgegenanschlag |
| 7 | Stössel | 17b | Ausnehmung |
| 7a | erste Ausnehmung | 17c | Verriegelungsglied |
| 7b | zweite Ausnehmung | | |
| 7c | erste Anschlagfläche | 18 | Verdrehsicherungshülse |
| 7d | zweite Anschlagfläche | | |
| | | | |
| 8 | Sperrhülse | 19 | Haltering |
| 8a | Verriegelungsglied | | |
| 8b | erste Ausnehmung | 20 | Kupplungsfeder / Klickfeder |
| 8c | zweite Ausnehmung | | |
| 8d | Haltefläche | 21 | Ring |
| 8e | Halteschnapper | | |
| | | 22 | Kupplungshülse |
| 9 | erste Feder/Aus schüttfeder | | |
| 100 | Dosisauswahlanschlag | H_{B} | Betätigungshub |
| | | H_{S} | Signalhub/erster Teilhub |
| 110 | erster Dosieranschlag | H_{K} | Startsignalhub |
| 111 | zweiter Dosieranschlag | H_{N} | Nadelschutzhub |
| 112 | Halteanschlag | H_{P} | Priminghub |
| | | | |
| H_{A} | Ausschütthub | L | Längsachse |

## Patentansprüche

1. Autoinjektor zur Verabreichung eines flüssigen Produkts, insbesondere Medikaments, der Autoinjektor umfassend:
- ein Gehäuse (2),
- einen Produktbehälterhalter (1) zur Halterung eines Produktbehälters (13) an dessen distalem Ende eine Nadel (13a) angeordnet ist und in welchem ein Kolben (13b) verschiebbar angeordnet ist,
- eine Nadelschutzhülse (3), die aus einer Ausgangsposition, in der das distale Ende der Nadelschutzhülse (3) distal über die Nadelspitze der Nadel (13a) steht, in das Gehäuse (2) verschiebbar ist, und
- einen Stössel (7) und eine Ausschüttfeder (9), wobei der Stössel (7) in dem Gehäuse (2) angeordnet ist und von der vorgespannten Ausschüttfeder (9) entlang einer Längsachse (L) in die distale Richtung verschiebbar ist, wobei die Verschiebung des Stössels (7) in die distale Richtung bewirkt, dass der Kolben (2) von dem Stössel (7) mitgenommen wird und das Produkt aus dem Produktbehälter (13) verdrängt, wobei
- die Nadelschutzhülse (3), wenn sie in das Gehäuse (2) bewegt wird, eine Sperrhülse (8) aus einer Position, in der sie verhindert, dass ein erstes Eingriffsglied (6a) eines Halteelementes (6) aus einer Ausnehmung (7a) des Stössels (7) bewegbar ist, in eine Freigabeposition verschiebt, wobei die Sperrhülse (8) in der Freigabeposition eine Bewegung des ersten Eingriffsglieds (6a) aus der Ausnehmung (7a) frei gibt, wodurch die Verschiebung des Stössels (7) in die distale Richtung für die Ausschüttung der eingestellten Dosis ermöglicht wird,
**dadurch gekennzeichnet dass**
- die Nadelschutzhülse (3), wenn sie in das Gehäuse (2) bewegt wird, eine Schalthülse (15) in die proximale Richtung verschiebt und die Schalthülse (15) während ihrer Verschiebung in die proximale Richtung an der Sperrhülse (8) anschlägt und diese mitnimmt.

2. Autoinjektor nach Anspruch 1, wobei durch die Verschiebung der Sperrhülse (8) eine erste Ausnehmung (8b) der Sperrhülse (8) entlang der Längsachse auf eine Position eines zweiten Eingriffsglieds (6b) des Halteelementes (6) verschoben wird, wodurch das zweite Eingriffsglied (6b) in die Ausnehmung (8b) der Sperrhülse (8) und das erste Eingriffsglied (6a) aus dem Eingriff mit der Ausnehmung (7a) des Stössels (7) verschoben werden.

3. Autoinjektor nach Anspruch 2, **dadurch gekennzeichnet, dass** das zweite Eingriffsglied (6b) in die Ausnehmung (8b) der Sperrhülse (8) und das erste Eingriffsglied (6a) aus dem Eingriff mit der Ausnehmung (7a) des Stössels (7) mit einer Bewegung quer zur Längsachse verschoben werden.

4. Autoinjektor nach einem der vorhergehenden Ansprüche, **dadurch gekennzeichnet dass** der Produktbehälterhalter (1) axialfest mit dem Gehäuse (2) verbunden ist, wobei die Nadel (13a) aus dem Gehäuse (2) über das distale Ende des Gehäuses (2) ragt.

5. Autoinjektor nach einem der vorhergehenden Ansprüche, **dadurch gekennzeichnet, dass** die Schalthülse (15) eine Ausnehmung (15a) aufweist, in welche ein an einem von der Sperrhülse (8) gebildeten Arm federnd angeordnetes Verriegelungsglied (8a) der Sperrhülse (8) eingreift.

6. Autoinjektor nach einem der vorhergehenden Ansprüche, **gekennzeichnet durch** eine Nadelschutzfeder (10), welche mit ihrem distalen Ende an der Schalthülse (15) abstützt und die Schalthülse (15) zumindest teilweise umgibt.

7. Autoinjektor nach Anspruch 6, **dadurch gekennzeichnet, dass** die Nadelschutzfeder (10) mit ihrem proximalen Ende auf ein Verschiebeglied (11) wirkt wodurch das Verschiebeglied (11) relativ zu dem Gehäuse (2) in die proximale Richtung bewegt werden kann und an einen feststehenden Teil des Autoinjektors anschlagen und dadurch ein taktiles und/oder akustisches Signal erzeugen kann, welches das Ende der Ausschüttung der eingestellten Dosis signalisiert.

## Claims

1. An autoinjector for administering a liquid product, in particular a drug, the autoinjector including:
- a housing (2),
- a product container holder (1) for holding a product container (13), at the distal end of which is arranged a needle (13a) and in which a piston (13b) is displaceably arranged,
- a needle protection sleeve (3) which is displaceable into the housing (2) from an initial position in which the distal end of the needle protection sleeve (3) is disposed distally beyond the needle tip of the needle (13a), and
- a plunger rod (7) and a dispensing spring (9), wherein the plunger rod (7) is arranged in the housing (2) and is displaceable by the prestressed dispensing spring (9) along a longitudinal axis (L) in the distal direction, wherein the displacement of the plunger rod (7) in the distal direction provides that the piston (2) is entrained by the plunger rod (7) and displaces the product from the product container (13), wherein
- the needle protection sleeve (3) when moved into the housing (2) displaces a locking sleeve (8) from a position in which it prevents a first engagement member (6a) of a holding element (6) being moveable out of a recess (7a) in the plunger rod (7) into a release position, wherein the locking sleeve (8) in the release position enables a movement of the first engagement member (6a) out of the recess (7a) whereby the displacement of the plunger rod (7) in the distal direction for dispensing the set dose is made possible,
**characterised in that**
- when the needle protection sleeve (3) is moved into the housing (2) it displaces a switching sleeve (15) in the proximal direction and the switching sleeve (15) during its displacement in the proximal direction abuts against the locking sleeve (8) and entrains same.

2. An autoinjector according to claim 1 wherein by the displacement of the locking sleeve (8) a first recess (8b) in the locking sleeve (8) is displaced along the longitudinal axis to a position of a second engagement member (6b) of the holding element (6) whereby the second engagement member (6b) is displaced into the recess (8b) in the locking sleeve (8) and the first engagement (6a) is displaced out of the engagement with the recess (7a) in the plunger rod (7).

3. An autoinjector according to claim 2 **characterised in that** the second engagement member (6b) is displaced into the recess (8b) in the locking sleeve (8) and the first engagement member (6a) is displaced out of the engagement with the recess (7a) in the plunger rod (7) with a movement transversely relative to the longitudinal axis.

4. An autoinjector according to one of the preceding claims **characterised in that** the product container holder (1) is axially fixedly connected to the housing (2), the needle (13a) projecting out of the housing (2) beyond the distal end of the housing (2).

5. An autoinjector according to one of the preceding claims **characterised in that** the switching sleeve (15) has a recess (15a) into which a locking member (8a) of the locking sleeve (8), that is arranged resiliently on an arm formed by the locking sleeve (8) engages.

6. An autoinjector according to one of the preceding claims **characterised by** a needle protection spring (10) which is supported with its distal end at the switching sleeve (15) and at least partially surrounds the switching sleeve (15).

7. An autoinjector according to claim 6 **characterised in that** the needle protection spring (10) acts with its proximal end on a displacement member (11) whereby the displacement member (11) can be moved relative to the housing (2) in the proximal direction and butts against a stationary part of the autoinjector and thereby can produce a tactile and/or acoustic signal signalling the end of dispensing of the set dose.

## Revendications

1. Auto-injecteur pour l'administration d'un produit liquide, en particulier d'un médicament, l'auto-injecteur comprenant :
- un boîtier (2),
- un support de récipient de produit (1) pour le support d'un récipient de produit (13), au niveau de l'extrémité distale duquel une aiguille (13a) est agencée et dans lequel un piston (13b) est agencé de manière mobile,
- une douille de protection d'aiguille (3) qui est mobile d'une position de départ, dans laquelle l'extrémité distale de la douille de protection d'aiguille (3) se trouve distalement au-dessus de la pointe de l'aiguille (13a), dans le boîtier (2), et
- un poussoir (7) et un ressort de distribution (9), dans lequel le poussoir (7) est agencé dans le boîtier (2) et est mobile par le ressort de distribution (9) précontraint le long d'un axe longitudinal (L) dans la direction distale, dans lequel le déplacement du poussoir (7) dans la direction distale mène à ce que le piston (2) soit entraîné par le poussoir (7) et refoule le produit hors du récipient de produit (13), dans lequel
- la douille de protection d'aiguille (3), lorsqu'elle est déplacée dans le boîtier (2), déplace une douille de verrouillage (8) d'une position, dans laquelle elle empêche qu'un premier élément de mise en prise (6a) d'un élément de retenue (6) puisse être déplacé hors d'un évidement (7a) du poussoir (7), dans une position de libération, dans lequel la douille de verrouillage (8) libère dans la position de libération un déplacement du premier élément de mise en prise (6a) hors de l'évidement (7a), par quoi le déplacement du poussoir (7) dans la direction distale est permis pour la distribution de la dose réglée,
**caractérisé en ce que**
- la douille de protection d'aiguille (3), lorsqu'elle est déplacée dans le boîtier (2), déplace une douille de commutation (15) dans la direction proximale et la douille de commutation (15) bute pendant son déplacement dans la direction proximale contre la douille de verrouillage (8) et l'entraîne.

2. Auto-injecteur selon la revendication 1, dans lequel par le déplacement de la douille de verrouillage (8) un premier évidement (8b) de la douille de verrouillage (8) est déplacé le long de l'axe longitudinal sur une position d'un second élément de mise en prise (6b) de l'élément de retenue (6), par quoi le second élément de mise en prise (6b) est déplacé dans l'évidement (8b) de la douille de verrouillage (8) et le premier élément de mise en prise (6a) hors de la mise en prise avec l'évidement (7a) du poussoir (7).

3. Auto-injecteur selon la revendication 2, **caractérisé en ce que** le second élément de mise en prise (6b) est déplacé dans l'évidement (8b) de la douille de verrouillage (8) et le premier élément de mise en prise (6a) est déplacé hors de la mise en prise avec l'évidement (7a) du poussoir (7) avec un déplacement transversal à l'axe longitudinal.

4. Auto-injecteur selon l'une des revendications précédentes, **caractérisé en ce que** le support de récipient de produit (1) est relié de manière fixe axialement au boîtier (2), dans lequel l'aiguille (13a) dépasse du boîtier (2) au-dessus de l'extrémité distale du boîtier (2).

5. Auto-injecteur selon l'une des revendications précédentes, **caractérisé en ce que** la douille de commutation (15) présente un évidement (15a), dans lequel un élément de verrouillage (8a), agencé de manière flexible au niveau d'un bras formé par la douille de verrouillage (8), de la douille de verrouillage (8) vient en prise.

6. Auto-injecteur selon l'une des revendications précédentes, **caractérisé par** un ressort de protection d'aiguille (10) qui s'appuie avec son extrémité distale contre la douille de commutation (15) et entoure au moins partiellement la douille de commutation (15).

7. Auto-injecteur selon la revendication 6, **caractérisé en ce que** le ressort de protection d'aiguille (10) agit avec son extrémité proximale sur un élément de déplacement (11), par quoi l'élément de déplacement (11) peut être déplacé par rapport au boîtier (2) dans la direction proximale et peut buter contre une partie fixe de l'auto-injecteur et ainsi générer un signal tactile et/ou acoustique qui signale la fin de la distribution de la dose réglée.
